# EUROPÄISCHE PATENTSCHRIFT

(11) **EP 2 060 189 B1**
(45) Veröffentlichungstag und Bekanntmachung des Hinweises auf die Patenterteilung: **17.10.2012**
(21) Anmeldenummer: 09152282.1
(22) Anmeldetag: 05.03.2004
(51) Int. Cl.: A23K 1/00, A23K 1/16, A23L 1/00, A23L 1/22, A61Q 19/00

(54) **Wirkstoffhaltige Adsorbate**
Adsorbate containing additive
Adsorbats contenant des agents actifs

(30) Priorität: 14.03.2003 DE 10311585
(43) Veröffentlichungstag der Anmeldung: 20.05.2009
(62) Teilanmeldung aus: 04717597.1
(73) Patentinhaber: BASF SE, 67056 Ludwigshafen (DE)
(72) Erfinder: Habich, Andreas, 67346 Speyer (DE); Hartleben, York, 67159 Friedelsheim (DE); Weller, Dietmar, 67067 Ludwigshafen (DE)

(56) Entgegenhaltungen:
- EP-A- 0 229 652
- US-A- 3 247 064
- US-A- 4 617 294
- CHAMBIN O ET AL: "Dry Adsorbed Emulsion: 1. Characterization of an Intricate Physicochemical Structure" JOURNAL OF THE PHARMACEUTICAL SCIENCES, Bd. 89, Nr. 8, 2000, Seiten 991-999, XP002521219

## Beschreibung

Wirkstoffhaltigen Zubereitungen sind in einer Vielzahl von technischen Anwendungen von Bedeutung, exemplarisch seien genannt Nahrungsmittel, Tierernährungsmittel oder kosmetische Mittel.

Allen Anwendungen gemeinsam ist das Problem, den jeweiligen Wirkstoff in einer technisch handhabbaren Formulierung bereit zu stellen und gleichzeitig die Stabilität der Wirkstoffe nicht zu beeinträchtigen. Bei der Stabilität der Wirkstoffe sind insbesondere die Stabilität in komplexeren Matrices, wie beispielsweise Tierfuttermitteln, insbesondere sogenannten "Prämixen" von großer Bedeutung. Weiterhin ist eine hohe Beladung der Formulierungen wünschenswert.

Im Stand der Technik hat es hierzu eine Vielzahl von Lösungsvorschlägen gegeben.

US 4,434,187 beschreibt Zubereitungen für die Fleischpökelung, welche neben dem Fleischbehandlungsmittel, Vitamin E, gegebenenfalls Lecithin sowie Silikapartikel enthalten. Die gemäß US 4,434,187 geeigneten Silikapartikel weisen eine durchschnittliche Partikelgröße von 1,5 bis 9 µm auf.

WO 97137546 A1 (EP 1 006 806) beschreibt frei fließende Zubereitungen, welche ein Mischung aus Fetten und optional einen Füllstoff enthalten. Als Füllstoff genannt wird Silika. Bei den dort beschriebenen Zubereitungen handelt es sich um physikalische Mischungen der Fette mit dem Füllstoff und keine Adsorbate.

WO 00/27362 A1 (EP 1 133 279) beschreibt trockene, frei-fließende Mischungen von flüssigen Tocopherolen, die Maisstärke und Silika enthalten. Das verwendete Silika hat eine Partikelgröße von 40 - 50 µm

WO 92/13531 A1 (EP 524 308) beschreibt Zubereitungen mit erhöhter Bioverfügbarkeit aus Vitamin E, einem Tensid Emulgator mindestens einem inerten Träger. Als inerte Träger genannt werden u.a. mikrokristalline Zellulose, Siliziumdioxid, Stärke. Die Mischungen aus Vitamin E und Tensid müssen einen HLB Wert von 7 bis 14 haben, um die erfindungsgemäß erforderliche erhöhte Bioverfügbarkeit aufzuweisen.

US 4,617,294 beschreibt frei fließende granuläre Silikapartikel mit einer Partikelgröße von 0,14 mm bis 0,84 mm und ihre Verwendung als Adsorbens für Vitamine. Die Verwendung von Stabilisatoren wird nicht diskutiert. US 4,617,294 beschreibt auf S. 3, Z.44-45, dass die öllöslichen Vitamine in einem Öl vorliegen können.

EP 0 062 225 B1 (BASF Corp.) beschreibt sprühgetrocknetes Vitamin E Pulver auf Basis von hydrolysierter Gelatine und Kaseinaten, welches mit 0,5 bis 2,0 Gew.-% Siliziumdioxid als Sprühtrocknungshiffsmittel oder Bepuderungsmittel hergestellt wird.

EP 0 326 026 B1 (BASF AG) beschreibt vitaminhaltige Dispersionen oder Emulsionen, welche durch übliche Trocknungsschritte in körnige oder pulverförmige Feststoffe überführt werden. Die Zubereitungen können Fließhilfsmittel, wie beispielsweise Siliciumdioxid sowie Füllstoffe, wie beispielsweise Stärke und Silikate enthalten.

JP 07-133491 beschreibt feste Zubereitungen, welche eine öllösliche Substanz enthalten. Sie werden erhalten durch Auftragen von flüssigen öllöslichen Verbindungen auf Silikate und anschließendes Verfestigen erhalten. Als geeignete Silikate werden spherische Partikel mit einer Partikelgröße von 20 bis 30 µm verwendet (Carplex Handelsprodukte der Fa. Shionogi & Co.). Die so erhältlichen Zubereitungen eignen sich als geruchsarme Darreichungsform insbesondere für Fischöle.

In EP 0 229 652 A2 werden Zubereitungen offenbart, die auf Tocopherolen als zu adsorbierender Komponente basieren.

Alle vorgeschlagenen technischen Lösungen sind jedoch hinsichtlich der Stabilität der Wirkstoffe, insbesondere in komplexeren Matrices, wie Lebensmittel und/oder Tierernährungsmitteln unbefriedigend. Hinzu kommt, dass es sich bei diesen Anwendungen in der Regel um Prozesse handelt, die besondere Anforderungen an die Handhabung (gute Fließeigenschaften, Staubarmut, geringe Kohäsivität, geringes Lufthaltevermögen etc.) sowie die weitere Verarbeitbarkeit (hohe Dosiergenauigkeit, keine Entmischung in den Mischungen mit anderen Bestandteilen) stellen. Insbesondere sollten die Formulierungen einfach in üblichen technischen Apparaturen herstellbar sein.

Die Aufgabe der vorliegenden Erfindung bestand demnach darin, wirkstoffhaltige Formulierungen bereitzustellen, die sich gut handhaben lassen und gleichzeitig eine hohe Stabilität der Wirkstoffe in den Zubereitungen gewährleisten. Aufgabe der vorliegenden Erfindung bestand darin, Zubereitungen zu Verfügung zu stellen, die es ermöglichen Wirkstoffe in komplexe Substrate, beispielsweise in Tierfuttermitteln oder Prämixen oder sonstigen Vermischungen für Tierfuttermitteln einzuarbeiten, ohne dass es zu Verklumpungen oder Entmischungserscheinungen oder sonstigen Anwendungs - oder Handhabungsnachteilen oder Verbackungen des Substrates kommt unter Erhalt der Stabilität des Wirkstoffes. Dabei ist von besonderem Interesse, dass die übrigen Inhaltsstoffe des komplexen Substrats durch die Zumischung nicht beeinflusst werden.

Von Bedeutung ist weiterhin, dass die Wirkstoffe in den Formulierungen rumengängig sind.

Die erfindungsgemäßen Aufgaben werden gelöst durch ein Verfahren zur Herstellung von für die menschliche oder tierische Ernährung sowie kosmetische Anwendungen geeigneten Adsorbaten gemäß Anspruch 1.

Adsorbate im Sinne der vorliegenden Erfindung sind insbesondere Zubereitungen, bei denen mindestens 10 Gew. %, insbesondere mindestens 20, bevorzugt mindestens 30, besonders bevorzugt mindestens 40, insbesondere mindestens 50 Gew. % der zu adsorbierenden Stoffe (Summe aller Bestandteile des Adsorbates ohne den Träger (C), d.h. üblicherweise mindestens Wirkstoff (A) und Stabilisator (B)) im inneren Porenvolumen des Trägers (C) vorliegen. Das innere Porenvolumen eines Trägers kann als Leervolumen nach der DPB (Dibutylphthalat) Methode DIN 53601 bestimmt werden.

Besonders bevorzugt sind Adsorbate, bei denen mindestens 60 Gew.-%, bevorzugt mindestens 70 Gew.-%, insbesondere mindestens 80 Gew.-% im inneren Porenvolumen des Trägers (C) vorliegen.

Gegenüber den in JP-07-133491 beschriebenen Zubereitungen zeichnen sich die Adsorbate der vorliegenden Erfindung durch bessere Handhabungs- und Anwendungsvorteile aus. Weiterhin sind die Adsorbate über übliche Wirbelschichttechniken und Mischertechniken zugänglich.

Die in US 4,617,294 beschriebenen vitaminhaltigen Zubereitungen enthalten keine Stabilisatoren im Sinne der vorliegenden Erfindung und weisen keine erhöhte Stabilität bei Lagerung oder in komplexen Matrices auf.

### Zu adsorbierende Stoffe (A)

Einsetzbar als Komponenten (A) sind konjugierte Octadecapolyensäuren, bei denen weniger als 5% des Fettsäureanteils 11,13-Octadecadiensäure-Isomere, 8,10-Octadecadiensäure-Isomere, cis-,cis-Octadecadiensäure-Isomere oder trans-, trans-Octadecadiensäure-Isomere oder Gemische dieser Isomeren sind.

Besonders bevorzugt als Komponenten (A) sind konjugierte Octadecapolyensäuren, bei denen weniger als 3% des Fettsäureanteils 11-,13-Octadecadiensäure-Isomere, 8-,10-Octadecadiensäure-Isomere, cis-,cis-Octadecadiensäure-Isomere oder trans-, trans-Octadecadiensäure-Isomere oder Gemische dieser Isomeren sind.

Besonders bevorzugt als Komponenten (A) sind konjugierte Octadecapolyensäuren, bei denen weniger als 1% des Fettsäureanteils 11-,13-Octadecadiensäure-Isomere, 8-,10- Octadecadiensäure-Isomere, cis-,cis-Octadecadiensäure-Isomere oder trans-, trans-Octadecadiensäure-Isomere oder Gemische dieser Isomeren sind.

In einer bevorzugten Ausführungsform der Erfindung werden, falls als zu adsorbierende Komponenten (A) ungesättigte Fettsäuren eingesetzt werden, als Stabilisatoren (B) mindestens eine Verbindung aus der Stoffklasse der Chelatoren (b-4) und mindestens eine Verbindung aus der Stoffklasse der Emulgatoren (b-2) eingesetzt.

Besonders bevorzugt sind hierbei Chelatoren (b-4) ausgewählt aus der Gruppe bestehend aus Salicylsäure, Stearylcitrat, Weinsäure und Zitronensäure.

Besonders bevorzugt sind hierbei Emulgatoren (b-2) ausgewählt aus der Gruppe bestehend aus
- Anlagerungsprodukte von 15 bis 60 Mol Ethylenoxid an Ricinusöl und/oder gehärtetes Ricinusöl; wie beispielsweise die unter dem Handelnamen Cremophor erhältlichen Produkte (BASF AG).
- Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen; und
- Anlagerungsprodukte von 2 bis 15 Mol Ethylenoxid an Ricinusöl und/oder gehärtetes Ricinusöl;

### Stabilisator (B)

Als Stabilisatoren werden Verbindungen eingesetzt, die ausgewählt sind aus der Gruppe bestehend aus **b-1) Glyceriden, b-2) Emulgatoren, b-3) Polysacchariden** und/oder **b-4) Chelatoren.**

Selbstverständlich können erfindungsgemäß sowohl Mischungen von Verbindungen aus den einzelnen Stoffklassen der Stabilisatoren (B) selbst, wie auch Mischungen von Verbindungen verschiedener Stoffklassen eingesetzt werden. Der Fachmann wählt den Stabilisator bzw. die Stabilisatorgemische dabei in Abhängigkeit von der zu adsorbierenden Komponente (A).

Besonders bevorzugt ist der Einsatz von mindestens einer Verbindung aus der Stoffklasse der Glyceride b-1) und mindestens einer Verbindung aus der Stoffklasse der Emulgatoren b-2).

Bevorzugt ist der Einsatz mindestens einer Verbindung aus der Stoffklasse der Glyceride b-1) und mindestens einer Verbindung aus der Stoffklasse der Polysaccharide b-3).

Bevorzugt ist der Einsatz mindestens einer Verbindung aus der Stoffklasse der Glyceride b-1) und mindestens einer Verbindung aus der Stoffklasse der Chelatoren b-4).

Bevorzugt ist der Einsatz mindestens einer Verbindung aus der Stoffklasse der Emulgatoren b-2) und mindestens einer Verbindung aus der Stoffklasse der Polysaccharide b-3).

Bevorzugt ist der Einsatz mindestens einer Verbindung aus der Stoffklasse der Emulgatoren b-2) und mindestens einer Verbindung aus der Stoffklasse der Chelatoren b-4).

Bevorzugt ist der Einsatz mindestens einer Verbindung aus der Stoffklasse Polysaccharide b-3) und mindestens einer Verbindung aus der Stoffklasse der Chelatoren b-4).

Besonders bevorzugt ist der Einsatz von mindestens einer Verbindung aus der Stoffklasse der Glyceride b-1), mindestens einer Verbindung aus der Stoffklasse der Emulgatoren b-2) sowie mindestens einer Verbindung aus der Stoffklasse der Polysaccharide b-3).

Besonders bevorzugt ist der Einsatz von mindestens einer Verbindung aus der Stoffklasse der Glyceride b-1), mindestens einer Verbindung aus der Stoffklasse der Emulgatoren b-2) sowie mindestens einer Verbindung aus der Stoffklasse der Chelatoren b-4).

Besonders bevorzugt ist der Einsatz mindestens einer Verbindung aus der Stoffklasse der Emulgatoren b-2), mindestens einer Verbindung aus der Stoffklasse der Polysaccharide b-3) sowie mindestens einer Verbindung aus der Stoffklasse der Chelatoren b-4).

Besonders bevorzugt ist der Einsatz mindestens einer Verbindung aus der Stoffklasse der Glyceride b-1), mindestens einer Verbindung aus der Stoffklasse der Polysaccharide b-3) sowie mindestens einer Verbindung aus der Stoffklasse der Chelatoren b-4).

### b-1 Glyceride

In einer bevorzugten Ausführungsform wird als Stabilisator (B) mindestens eine Verbindung eingesetzt, die ausgewählt ist aus der Stoffklasse der Glyceride (b-1).

Bevorzugt werden als Stabilisatoren mindestens 2, insbesondere mindestens 3 Verbindungen ausgewählt aus der Stoffklasse der Glyceride (b-1) eingesetzt.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Gylceridgemisch kann weitere Zusätze, z.B. freie Fettsäuren, enthalten.

Das eingesetzte Glycerid kann ein synthetisches oder natürlich vorkommendes Glyceridöl oder ein Derivat davon sein. Unter "Glycerid" werden auch synthetische oder natürlich vorkommende Fettsäureester und/oder Glyceride enthaltende Öle und Fette verstanden.

Unter einem "Glycerid" werden ferner vom Glycerin abgeleitete Derivate verstanden. Insbesondere Derivate, bei denen die Fettsäurezusammensetzung der natürlich vorkommenden nicht konjugierten oder gesättigten Glyceride sich nicht wesentlich geändert hat, sind umfasst.

Bevorzugt sind synthetische oder natürliche Glyceride, die Acylreste mit 1 bis 22 Kohlenstoffatomen, bevorzugt mit 12 bis 18 Kohlenstoffatomen enthalten. Besonders bevorzugt sind natürliche Öle und Fette, die gesättigte und/oder einfach oder mehrfach ungesättigte Acylreste mit mehr als 12, insbesondere mehr als 16 Kohlenstoffatomen und weniger als 22 Kohlenstoffatome enthalten, bevorzugt von 18 bis 20 Kohlenstoffatome.

Unter dem Begriff "Öl" oder "Fett" wird ein Fettsäuregemisch verstanden, das gesättigte, ungesättigte, geradkettige und/oder verzweigte veresterte Fettsäure(n) enthält. Die Fettsäuren haben dabei üblicherweise 1 bis 22, insbesondere 12 bis 18, bevorzugt 12 bis 16 C-Atome.

Das Öl oder Fett kann verschiedene andere gesättigte oder ungesättigte Fettsäuren, enthalten. Insbesondere kann je nach Herstellungsverfahren der Anteil der verschiedenen Fettsäuren in dem Öl oder Fett schwanken. Fettsäureester sind ebenfalls von der erfindungsgemäßen Formulierung umfasst, insbesondere Fettsäureester, die bei der Herstellung von Öl aus pflanzlichem Material entstehen. Beispielsweise seien genannt C1 bis C12 Alkylester von Fettsäuren. Vorzugsweise liegen die Fettsäureester als Glycerid, insbesondere als Triglycerid vor.

Als pflanzliches oder tierisches Ausgangsmaterial sind z.B. Olivenöl, Kokosöl, Kokosfett, Sesamöl, Reiskeimöl, Bambusöl, Bambusfett, Sonnenblumenöl, Rapsöl, Fischöl, Sojaöl, Palmöl, Färberdistelöl, Leinöl, Weizenkeimöl, Erdnussöl, Baumwollsaatöl, Maiskeimöl, Schweinefett, Rinderfett, Geflügelfett, Milchfett, TungÖl oder Sheaöl oder ein Derivat oder eine Mischung davon einsetzbar. Einsetzbar sind ebenfalls hydrierte oder teilhydrierte Öle und Fette.

Folgende Tabelle stellt geeignete Glyceride zusammen:

| Glycerid Handelsname [Hersteller] | Zusammensetzung | Schmelzbereich | CAS-Nr. / INCI |
|---|---|---|---|
| Shoguwar 41 [Aarhuus Olive] | Hydriertes Sojaöl | ca. 41 °C | |
| Rucawar FH [Aarhuus Olie] | Hydriertes Rapsöl | ca. 61 °C | |
| Precirol ATO 5 [Gattefosse] | Mono-, Di-, Triglyceride der Palmitin- und Stearinsäure | ca. 60 °C | 555-44-2 |
| | | | 555-43-1 |
| Biogapress Vegetal ATO [Gattefosse] | Ester der Palmitin- und Stearinsäure | 60 - 65 °C | |
| Compritol 888 [Gattefosse] | Mono-, Di-, Triester der Behen - und Arachidonsäure | ca. 75 °C | 94201-62-4 |
| | | | 18641-57-1 |
| | | | 30233-64-8 |
| Compritol E [Gattefosse] | Mono-, Di-, Triglyceride der Behensäure | 75 - 80 °C | 18641-57-1 |
| Vegeol CO-52S [Aarhuus Olie] | Mischung aus Palmitinsäure und Stearinsäure | 60 - 65 °C | |
| Palmowar FH [Aarhuus Olie] | Mischung aus Palmitinsäure und Stearinsäure | 60 - 65 °C | |
| Colzawar 46 [Aarhuus Olie] | Mischung aus Palmitinsäure und Stearinsäure | 50 - 55 °C | |
| Vegeol PR-267 [Aarhuus Olie] | Mischung aus Colzawar 46 und Rucawar FH | ca. 70 °C | |
| Vegeol PR-269 [Aarhuus Olie] | Mischung aus Vegeol CO-52S und Palmowar FH | 60 - 65 °C | |
| Cutina CP [Henkel / Cognis] | Synthetisches Cetylpalmitat | 46 - 51 °C | 95912-87-1 |
| Edenor NHTi [Cognis] | Hydrierter Rindertalg | 56-61 °C | 67701-27-3 |
| Edenor NHTi V [Cognis] | Pflanzenfett mit ähnlicher C-Kettenverteilung wie hydrierter Rindertalg | 57-61 °C | 67701-27-3 |

### Emulgatoren b-2

In einer bevorzugten Ausführungsform wird als Stabilisator (B) mindestens eine Verbindung ausgewählt aus der Stoffklasse der Emulgatoren (b-2) eingesetzt.

Bevorzugt werden als Stabilisatoren mindestens 2, insbesondere mindestens 3 Verbindungen ausgewählt aus der Stoffklasse der Emulgatoren (b-2) eingesetzt.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl; wie beispielsweise die unter dem Handelnamen Cremophor erhältlichen Produkte (BASF AG).
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEGalkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglycose und Polyolen, vorzugsweise Glycerin oder Polyglycerin sowie
(13) Polyalkylenglycole

Die Anlagerungsprodukte von Ethylenoxid und/oder von Propylenoxid an Fettalkohole, Fettsäuren, Alkylphenole, Glycerinmono- und -diester sowie Sorbitanmono- und - diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE-PS 2024051 als Rückfettungsmittel für kosmetische Zubereitungen bekannt. C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidesters gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solcher oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH - oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Emulgatoren können weiterhin eingesetzt werden Glycerophospholipide und Glyceroglycolipide. Bevorzugt sind hier die Glycerophospholipide wie Lecithin (Phosphatidylcholin), Cardiolipin, Phosphatidylglycerin, Phosphatidylserin und Alkylacylglycerophospholipide wie das Plasmalogen

Eine Zusammenstellung aller Emulgatoren, die insbesondere für kosmetische Anwendungen geeignet sind, findet sich im Handbook of Cosmetic Ingredients der CTFA (Cosmetic, Toiletry and Fragrance Association) und "Emulsifiers" bzw. in der CTFA online Datenbank (www.ctfa.org).

Insbesondere geeignet sind die als Lebensmittel- Emulgatoren bekannten Diacylglyceride sowie ihre Mischungen wie beispielsweise die Lebensmittelemulgatoren E 472a, E 472b, E 472 e oder E 442. Geeignet sind weiterhin alle in "Emulgatoren für Lebensmittel" hrsg. G. Schuster, Springer Verlag 1985 auf S.55 bis S. 190 genannten Emulgatoren. Hiervon besonders geeignet sind die in Tabelle 1 (S. 57-60) genannten Emulgatoren.

Bevorzugt sind Emulgatoren mit den EWG-Nr. E 322 Lecithine,
E432 Polyoxyethlyene(20)sorbitanmonolaurate, E433 Polyoxyethlyene(20) sorbitanmonooleat, E434 Polyoxyethlyene(20)sorbitanmonopalmitat,
E435 Polyoxyethlyene(20)sorbitanmonostearat, E436 Polyoxyethlyene(20) sorbitanmonotristearat, E 470, E 471 Mono- und Diglycerid von Fettsäuren, E 472a, E 472b, E 472c, E472d, E 472e, E 473 Sucroseester von Fettsäuren, E 474, E 475, E476, E 477, E 481, E 482 Calciumstearoyl-2-lactylat, E 483 Stearyltartrat sowie E 493, E 494, E 495, E491, E492 und E 498.

Die folgende Tabelle stellt besonders geeignete Emulgatoren zusammen

| Emulgator Handelname [Hersteller] | Charakterisierung |
|---|---|
| Metarin FM [Degussa] | Sojalecithin Mischung aus unpolaren Triglyceriden und polaren Phospho- und Glycolipiden - pflanzliches Öl, Emulqator E 322 |
| Cremophor WO 7 [BASF] | Hydriertes Rizinusöl, ethoxyliert CAS - Nr.: 61788-85-0 INCI-Name: PEG-7 Hydrogenated Castor Oil |
| Cremophor CO 40 [BASF] | Hydriertes Rizinusöl, ethoxyliert CAS - Nr.: 61788-85-0 INCI-Name: PEG-40 Hydrogenated Castor Oil |
| Imwitor 600 [Condea Chemie] | Polyglycerin-Polyricinoleat (PGPR) Emulgator E 476 |
| Grinsted PGPR 90 [Danisco] | Polyglycerin-Polyricinoleat (PGPR) Emulqator E 476 |
| Emulan A [BASF AG] | Ölsäureethoxylat CAS Nr. 9004-96-0 |
| Cremophor GO 32 [BASF Corp.] | Polyglyceryl-3-oleat CAS Nr. 9007-48-1 (Generic) 33940-98-6 |
| Cremophor GS 32 [BASF Corp.] | Polyglyceryl-3-distearat CAS Nr. 9009-32-9 (Generic) 94423-19-5 |
| Lutensol TO 8 [BASF AG] | C₁₃-Oxoalkoholethoxylate CAS Nr. 69011-36-5 |
| Plantacare E 6000 | Caprylyl/Capryl Glucoside |
| [Cognis) | |
| Pluronic™PE [BASF AG] | PO/EO Blockpolymerisate |

Besonders bevorzugt sind Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe.

Besonders bevorzugt sind EO/PO Blockpolymerisate, wie sie beispielsweise unter dem Handelsnamen Pluronic™PE (BASFAktiengesellschaft) erhältlich sind.

Besonders bevorzugt sind Anlagerungsprodukte von 2 bis 15 Mol Ethylenoxid an Ricinusöl und/oder gehärtetes Ricinusöl, wie sie beispielsweise unter dem INCI Namen PEG-7 Hydrogenated Castor Oil erhältlich sind.

Besonders bevorzugt sind Anlagerungsprodukte von 15 bis 60 Mol Ethylenoxid an Ricinusöl und/oder gehärtetes Ricinusöl; wie beispielsweise unter dem INCI Namen PEG-40 Hydrogenated Castor Oil erhältlichen Produkte.

Besonders geeignet sind Polyol- und insbesondere Polyglycerinester, wie z.B. Polyglycerinpolyricinoleat, Polyglycerinpoly-12-hydroxystearat oder Polyglycerindimerat, wie sie unter den INCI Namen Polyglyceryl-3-Distearate oder Polyglyceryl-3 Oleate erhältlich sind. Ebenfalls geeignet sind Gemische von Verbindungen aus mehreren dieser Substanzklassen.

In einer bevorzugten Ausführungsform der Erfindung werden als Stabilisatoren (B) mindestens eine Verbindung aus der Stoffklasse der Chelatoren (b-4) und mindestens eine Verbindung aus der Stoffklasse der Emulgatoren (b-2) eingesetzt.

### b-3) Polysaccharide

In einer bevorzugten Ausführungsform wird als Stabilisator (B) mindestens eine Verbindung ausgewählt aus der Stoffklasse der Polysaccharide (b-3) eingesetzt.

Bevorzugt werden als Stabilisatoren mindestens 2, insbesondere mindestens 3 Verbindungen ausgewählt aus der Stoffklasse der Polysaccharide (b-3) eingesetzt.

Werden als Träger C) Polysaccharide eingesetzt, so werden als Stabilisatoren (B) von (C) verschiedene Polysaccharide eingesetzt.

Polysaccharide (synonym Glykane) ist die Sammelbezeichnung für makromolekulare Kohlenhydrate, deren Molekül aus einer großen Zahl (mindestens >10, bevorzugt > 20, insbesondere > 50) glykosidisch miteinander verknüpfter Monosaccharid Moleküle (Glykose) bestehen.

Als Polysaccharide seien genannt die Homoglykane Stärke, Glykogen, Guaran, Amylopektin sowie Cellulose, Amylose, Dextran u. Tunicin (Polyglucosane, Glucane), Inulin als Polykondensat der D-Fructose (Polyfructosan, Fructan), Chitin, Alginsäure.

Weiterhin geeignet sind Heteropolysaccharide (Heteroglykane) aus verschiedenartigen Monomer-Einheiten. Bekannte Heteroglykane sind Pektine, Mannane, Galactane, Xylane u.a. Polyosen, ferner Chondroitinsulfate, Heparin, Hyaluronsäure u.a. Glykosaminoglykane.

Geeignet sind beispielsweise Maltodextrine, wie sie kommerziell erhältlich sind unter dem Namen N-Zorbit M (Maltodextrine aus Tapiokastärke, National Starch).

Geeignet sind des weiteren Stärkederivate, beispielsweise wie Stärke-Octenylsuccinat, kommerziell erhältlich unter dem Namen HI-CAP 100 von National Starch.

### b-4) Chelatoren

In einer bevorzugten Ausführungsform wird als Stabilisator (B) mindestens eine Verbindung ausgewählt aus der Stoffklasse der Chelatoren (b-4) eingesetzt.

Bevorzugt werden als Stabilisatoren mindestens 2, insbesondere mindestens 3 Verbindungen ausgewählt aus der Stoffklasse der Chelatoren (b-4) eingesetzt.

Geeignet Chelatoren sind prinziell alle als Chelatoren für Lebensmittel und Tierfuttermittel zugelassenen Verbindungen. Exemplarisch seien genannt: Na-, K-, Ca-Salze der Essigsäure, Citronensäure sowie die Na-, K-, Ca-Salze der Citronensäure, Citronensäuremonoisoproplyester, Monoglyceridester, Triethylester sowie Monostearlyester der Citronensäure, EDTA, Na-, K-, Ca-Salze von EDTA, Na-, K-, Ca-Salze der Gluconsäure, Oxystearin, Orthophosphorsäure sowie die Na-, K-, Ca-Salze der, Orthophosphorsäure, Na-Salz der Pyrophosphorsäure, Na-Salz der Triphosphorsäure, Na- und Ca-Salze der Hexametaphosphorsäure, Ca-Salz der Phytinsäure, Sorbit, Weinsäure, Salicylsäure, sowie das Na-Salz der Thioschwefelsäure.

Insbesondere geeignet sind Weinsäure, Salicylsäure sowie Monostearlyester der Citronensäure.

Geeignet Chelatoren sind beschrieben in Thomas E. Furia, "Sequestrants in Food" CRC Handbook of Food Additives, 2nd edition, Volume I, CRC Press, Boca Raton, ISBN 0-8493-0542-X, Chapter 6, S. 271-280. Insbesondere geeignet sind die dort auf S. 275 -277 genannten Chelatoren.

In einer bevorzugten Ausführungsform der Erfindung ist das Gewichtsverhältnis von Stabilisator (B) zu adsorbierender Komponenten (A) kleiner gleich 10, insbesondere kleiner gleich 5, insbesondere kleiner gleich 1, insbesondere kleiner gleich 0,8, insbesondere kleiner gleich 0,5, bevorzugt kleiner gleich 0,3 ist.

Ist der Stabilisator (B) ausgewählt aus der Gruppe der Glyceride (b-1) und/oder Polysaccharide (b-3), ist bevorzugt ein Gewichtsverhältnis von Stabilisator (B) zu adsorbierender Komponenten (A) kleiner gleich 10, insbesondere kleiner gleich 5, insbesondere kleiner gleich 1, insbesondere kleiner gleich 0,8.

Ist der Stabilisator (B) ausgewählt aus der Gruppe der Emulgatoren (b-2) und/oder Chelatoren (b-4), ist bevorzugt ein Gewichtsverhältnis von Stabilisator (B) zu adsorbierender Komponenten (A) kleiner gleich 0,8, besonders kleiner gleich 0,5, insbesondere kleiner gleich 0,3, bevorzugt kleiner gleich 0,1.

### Träger (C)

Als Träger (C) wird Kieselsäure eingesetzt. Solche Produkte sind kommerziell erhältlich, z.B. Sipernat® 22 oder Sipernat® 2200 (Degussa); Tixosil® 38 X oder Tixosil® 68 (Rhodia) oder Zeofree 5170 (Huber).

Besonders bevorzugt sind hydrophile Kieselsäuren.

Erfindungswesentlich ist die mittlere Partikelgröße des Trägers (C) von mindestens 80 µm, bevorzugt von mindestens 100 µm, bevorzugt von mindestens 200 µm, insbesondere mindestens 300 µm, bevorzugt mindestens 400 µm.

In einer bevorzugten Ausführungsform werden die Träger eingesetzt, deren mittlere Partikelgröße unter 1000 µm, insbesondere unter 800 µm liegt.

Die mittlere Partikelgröße wird dabei als [D4,3] Wert angegeben. Bestimmt werden kann diese auf einem Mastersizer S der Fa. Malvern Instruments GmbH, Serial Number 32734-08. Zur Beschreibung der Breite der Partikelgrößenverteilung wurden die D(v,0.1), D(v,0.5) und D(v,0.9) bestimmt sowie die mittlere Partikelgröße der Verteilung [D4,3] angegeben.

Die mittlere Partikelgröße [D4,3) wird gemäß des Mastersizer Reference Handbook, Preliminary Manual Chapter 2, page 22-23, Malvem Instruments Ltd zum Mastersizer S Serial No. 32734-08, Certificate No P 1261, Mai 1995 angegeben.
D[4,3] wird als "the volume weighted mean diameter" angegeben.

Die so bestimmten mittlere Partikelgrößen [D4,3] betragen z.B. für Sipernat® 22: 136 µm, Sipernat® 2200: 364 µm, Tixosil® 38 X: 265 µm, Tixosil® 68: 302 µm oder Zeofree 5170: 287 µm.

Der Träger (C) kann in der erfindungsgemäßen Formulierung, bezogen auf das Gesamtgewicht des Adsorbats, in einem Anteil von über 10 Gew. %, insbesondere über 20 Gew. %, bevorzugt über 30 Gew. % vorliegen. Üblicherweise liegt der Träger in einem Bereich von 10 bis 85 Gew.-%, vorzugsweise etwa 20 bis 85 Gew.%, bezogen auf das Gesamtgewicht des Adsorbats vor. Diese Werte beziehen sich auf das unbeschichtete stabilisierte Adsorbat.

### Beschichtungsmittel D)

In einer bevorzugten Ausführungsform der Erfindung wird das Adsorbat beschichtet. Als geeignete Beschichtungsmittel seien genannt:
a) Polyalkylenglycole, insbesondere Polyethylenglycolen mit einem zahlenmittleren Molekulargewicht von etwas 400 bis 15 000, wie z.B. 400 bis 10 000;
b) Polyalkylenoxid-Polymeren oder -Copolymeren mit einem zahlenmäßigen Molekulargewicht von etwa 4000 bis 20 000, insbesondere Blockcopolymere von Polyoxyethylen und Polyoxypropylen;
c) Substituierte Polystyrole, Maleinsäurederivate und Styrolmaleinsäurecopolymere;
d) Polyvinylpyrrolidone mit einem zahlenmittleren Molekulargewicht von etwas 7 000 bis 1 000 000;
e) VinylpyrrolidonNinylacetat-Copolymere mit einem zahlenmittleren Molekulargewicht von etwa 30 000 bis 100 000;
f) Polyvinylalkohol mit einem zahlenmittleren Molekulargewicht von etwa 10 000 bis 200 000, Polyphthalsäurevinylester;
g) Hydroxypropylmethylcellulose mit einem zahlenmittleren Molekulargewicht von etwa 6000 bis 80 000;
h) Alkyl(meth)acrylat-Polymere und -Copolymere mit einem zahlenmittleren Molekulargewicht von etwa 100 000 bis 1 000 000, insbesondere Ethylacrylat/Methylmethacrylat-Copolymere und Methacrylat/Ethylacrylat-Copolymere;
i) Polyvinylacetat mit einem zahlenmittleren Molekulargewicht von etwas 250 000 bis 700 000 ggfls. Stabilisiert mit Polyvinylpyrrolidon;
j) Polyalkylenen, insbesondere Polyethylenen;
k) Phenoxyessigsäure-Formaldehyd-Harz;
l) Cellulosederivate, wie Ethylcellulose, Ethylmethylcellulose, Methylcellulose, Hydroxypropylcellulose, Hydroxypropylmethlycellulose, Carboxymethylcellulose, Celluloseacetatphthalat;
m) tierische, pflanzliche oder synthetische Glyceride;
n) Tierische, pflanzliche oder synthetische Wachse oder chemisch modifizierte tierische, pflanzliche Wachse wie Bienenwachs, Candelillawachs, Carnaubawachs, Montanesterwachs und Reiskeimölwachs, Walrat, Lanolin, Jojobawachs, Sasolwachs, Japanwachs oder Japanwachsersatz;
o) Tierische und pflanzliche Proteine wie z.B. Gelatine, Gelatinederivate, Gelatineersatzstoffe, Casein, Molke, Keratin, Sojaprotein; Zein und Weizenprotein;
p) Mono- und Disaccharide, Oligosaccharide, Polysaccharide, z.B. Stärken, modifizierte Stärken sowie Pektine, Alginate, Chitosan, Carrageene;
q) pflanzliche Öle, z.B. Sonnenblumen-, Distel-, Baumwollsaat-, Soja-, Maiskeim-, Oliven-, Raps(samen)-, Lein-, Ölbaum-, Kokos-, (Öl)Palmkernöl und Palmöl;
r) synthetische oder halbsynthetische Öle, z.B. mittelkettige Triglyceride oder Mineralöle;
s) tierische Öle wie z.B. Hering-, Sardine- und Walöl;
t) gehärtete (hydrierte oder teilhydrierte) Öle/Glyceride wie z.B. von den oben genannten, insbesondere hydriertes Palmöl, hydriertes Baumwollsaatöl, hydriertes Sojaöl;
u) Lackcoatings wie z.B. Terpene, insbesondere Schellack, Tolubalsam, Perubalsam, Sandarak, und Silikonharze;
v) Fettsäuren, sowohl gesättigte als auch einfach und mehrfach ungesättigte C6 bis C24-Carbonsäuren;
w) Kieselsäuren.

Die genanntem Beschichtungsmittel können auch in Mischungen untereinander eingesetzt werden.

Als Beispiele für geeignete Polyalkylenglykole a) sind zu nennen: Polypropylenglykole und insbesondere Polyethylenglykole unterschiedlicher Molmasse, wie z. B. PEG 4000 oder PEG 6000, erhältlich von der BASF AG unter den Handelsnamen Lutrol E 4000 und Lutrol E 6000.

Als Beispiele für obige Polymere b) sind zu nennen: Polyethylenoxide und Polypropylenoxide, Ethylenoxid/Propylenoxid-Mischpolymere sowie Blockcopolymere, aufgebaut aus Polyethylenoxid- und Polypropylenoxidblöcken, wie z. B. Polymere, die von der BASF AG unter der Handelsbezeichnung Lutrol F68 und Lutrol F127 erhältlich sind.

Von den Polymeren a) und b) können vorzugsweise hochkonzentrierte Lösungen von bis zu etwa 50 Gew.-%, wie z. B. etwa 30 bis 50 Gew.%, bezogen auf das Gesamtgewicht der Lösung, vorteilhaft eingesetzt werden.

Als Beispiele für obige Polymere d) sind zu nennen: Polyvinylpyrrolidone, wie sie beispielsweise von der BASF AG unter dem Handelsnamen Kollidon oder Luviskol vertrieben werden. Von diesen Polymeren können hochkonzentrierte Lösungen mit einem Feststoffanteil von etwa 30 bis 40 Gew.-%, bezogen auf das Gesamtgewicht der Lösung, vorteilhaft eingesetzt werden.

Als Beispiel für oben genannte Polymere e) ist zu nennen: ein VinylpyrrolidonNinylacetat-Copolymeres, welches von der BASF AG unter der Handelsbezeichnung Kollidon VA64 oder Kollicoat SR vertrieben wird. Von diesen Copolymeren können hochkonzentrierte Lösungen von etwa 30 bis 40 Gew.%, bezogen auf das Gesamtgewicht der Lösung, besonders vorteilhaft eingesetzt werden.

Als Beispiel für obige Polymere f) sind zu nennen: Produkte, wie sie beispielsweise von der Fa. Hoechst unter der Handelsbezeichnung Mowiol vertrieben werden. Von diesen Polymeren können Lösungen mit einem Feststoffanteil im Bereich von etwa 8 bis 20 Gew.-% vorteilhaft eingesetzt werden.

Als Beispiele für geeignete Polymere g) sind zu nennen: Hydroxypropylmethylcellulosen, wie sie z. B. vertrieben werden von Shin Etsu unter dem Handelsnamen Pharmacoat.

Als Beispiele für oben genannte Polymere h) sind zu nennen: Alkyl(meth)acrylat-Polymere und -Copolymere, deren Alkylgruppe 1 bis 4 Kohlenstoffatome aufweist. Als konkrete Beispiele für geeignete Copolymere sind zu nennen: Ethylacrylat/Methylmethacrylat-Copolymere, welche beispielsweise unter den Handelsnamen Kollicoat EMM 30D von der BASF AG oder unter dem Handelsnamen Eutragit NE 30 D von der Fa. Röhm vertrieben werden; sowie Methacrylat/Ethylacrylat-Copolymere, wie sie beispielsweise unter dem Handelsnamen Kollicoat MAE 30DP von der BASF AG oder unter dem Handelsnamen Eutragit 30/55 von der Fa. Röhm vertrieben werden. Derartige Copolymere können beispielsweise als 10 bis 40 gew.%ige Dispersionen erfindungsgemäß verarbeitet werden.

Als Beispiele für obige Polymere i) sind zu nennen: Polyvinylacetat-Dispersionen, welche mit Polyvinylpyrrolidon stabilisiert sind und beispielsweise unter der Handelsbezeichnung Kollicoat SR 30D von der BASF AG vertrieben werden (Feststoffgehalt der Dispersion etwa 20 bis 30 Gew.-%).

Fette, z.B. solche tierischen, pflanzlichen oder synthetischen Ursprungs; Als Beispiele für tierische Fette m) seien Fette von Schwein, Rind und Gänsen genannt, geeignet ist beispielweise Rindertalg. Ein geeignetes Rindertalg ist unter dem Handelsnamen Edenor NHIT-G (CAS Nr. 67701-27-3) der Fa. Cognis erhältlich.

Weitere Beschichtungsmittel sind Gelatine, z.B. vom Rind, vom Schwein, vom Fisch.

Weitere Beschichtungsmittel sind Wachse, z.B. pflanzliche Wachse, wie z.B. Candelilawachs, Carnaubawachs, Reiskeimölwachs, Japanwachs oder Japanwachsersatz (erhältlich unter dem Handelsnamen Japanwachsersatz 2909, Kahl Wachsraffinerie) etc.; synthetische Wachse, wie Cetylpalmitat (erhältlich unter dem Handelsnamen Cutina CP , CAS 95912-87-1 der Fa. Cognis), tierische Wachse, wie z.B. Lanolin, Bienenwachs, Schellackwachs, Walrat sowie chemisch modifizierte Wachse wie Jojobawachs, Sasolwachs, Montaneesterwachs.

Prinzipiell sind auch andere Beschichtungen aus der Lösung vorstellbar: z.B. Zuckercoating.

Ebenso können pflanzliche Öle q), z.B. Sonnenblumen-, Distel-, Baumwollsaat-, Soja-, Maiskeim- und Olivenöl, Raps-, Lein-, Ölbaum-, Kokosnuss-, (Öl)Palmkern- und (Öl)Palmöl; in Betracht kommen. Geeignete Palmöle sind beispielsweise unter dem Handelsnamen Vegeol PR 265 der Fa. Aarhus Oliefabrik erhältlich. Geeignete Raps(samen)öle sind unter dem Handelsnamen Vegeol PR 267 der Fa. Aarhus Oliefabrik erhältlich. Palmkernöl ist unter dem Handelsnamen Tefacid Palmic 90 (CAS Nr. 57-10-3) der Fa. Karlshamns erhältlich.

Ebenso können halbsynthetische Öle r), z.B. mittelkettige Triglyceride oder Mineralöle und/oder tierische Öle s), z.B. Hering-, Sardinen- und Walöle in Betracht kommen.

In einer bevorzugten Ausführungsform werden als Beschichtungsmittel eingesetzt hydrierte pflanzliche Öle t) einschließlich Triglyceride, wie z.B. hydrierte Baumwollsamen-, Mais-, Erdnuss-, Sojabohnen-, Palm-, Palmkern-, Babassu-, Sonnenblumen - und Färberdistelöle. Bevorzugte hydrierte pflanzliche Öle umfassen hydriertes Palmöl, Baumwollsamenöl und Sojabohnenöl. Das am meisten bevorzugte hydrierte pflanzliche Öl ist hydriertes Sojabohnenöl. Andere von Pflanzen und Tieren stammende Fette und Wachse sind ebenfalls geeignet.

Die bevorzugt eingesetzten hydrierten pflanzlichen Öle können in verschiedenen polymorphen Formen vorliegen, dieses sind die α-, β- und β'-Form. In einer besonders bevorzugten Ausführungsform der vorliegenden Erfindung werden hydrierte pflanzliche Öle eingesetzt, die überwiegend in der β- und β'- Form vorliegen, insbesondere solche, die überwiegend in der β-Form vorliegen. Unter dem Begriff "überwiegend" ist zu verstehen, dass mindestens 25 %, insbesondere mindestens 50 %, bevorzugt mindestens 75% der Kristalle in der bevorzugten polymorphen Form vorliegen.

Besonders bevorzugt ist der Einsatz von hydriertem Sojabohnenöl mit einem Anteil von über 50 %, insbesondere über 75 %, bevorzugt über 90 % β und/oder β'-Form.

Für die Beschichtung kann z.B. eine möglichst hochkonzentrierte, noch sprühfähige Flüssigkeit, wie z. B. eine bis 50 gew.-%ige wässrige oder nichtwässrige Lösung oder Dispersion eines oder mehrerer der genannten Beschichtungsmaterialien eingesetzt werden. Ebenso könne pulverförmige Beschichtungsmaterialien eingesetzt werden.

Bevorzugte Beschichtungsmittel umfassen hydrierte pflanzliche Öle einschließlich Triglyceride, wie z.B. hydrierte Baumwollsamen-, Mais-, Erdnuss-, Sojabohnen-, Palm-, Palmkern-, Babassu-, Sonnenblumen- und Färberdistelöle. Bevorzugte hydrierte pflanzliche Öle umfassen hydriertes Palmöl, Baumwollsamenöl und Sojabohnenöl. Das am meisten bevorzugte hydrierte pflanzliche Öl ist hydriertes Sojabohnenöl. Andere von Pflanzen und Tieren stammende Fette und Wachse sind ebenfalls geeignet.

### Weitere Stoffe (E)

In einer weiteren Ausführungsform der Erfindung können die Adsorbate weitere Stoffe enthalten, beispielsweise seien genannt Antioxidantien, Konservierungsmittel, Bindemittel und /oder Geschmacksstoffe enthalten.

Antioxidationsmittel sind z.B. vorteilhaft, um die Doppelbindungen der Fettsäuren vor Oxidation zu schützen. Jedoch ist auch die allgemeine gesundheitsfördernde Wirkung von Antioxidationsmitteln bekannt. So werden in der Tierernährung als Antioxidationsmittel vorzugsweise Ethoxyquin, Ascorbinsäure, t-Butylhydroxytoluol, t-Butylhydroxyanisol, Ascorbylpalmitat verwendet, ansonsten werden auch gamma- und alpha-Tocopherole, Tocotrienol, Rosmarinextrakt, Isoflavone und Carotinoide und natürlich vorkommende Polyphenole, z. B. Flavonoide eingesetzt.

In einer bevorzugten Ausführungsform der Erfindung werden als weitere Stoffe (E) Antioxidantien eingesetzt, falls als zu adsorbierende Komponenten (A) öllösliche Verbindungen eingesetzt werden. Insbesondere falls es sich bei der zu adsorbierenden Komponenten um öllösliche Vitamine und/oder ungesättigte Fettsäuren handelt.

In einer bevorzugten Ausführungsform der Erfindung werden als weitere Stoffe (E) Antioxidantien eingesetzt, falls als Stabilisator (B) eine Verbindung aus der Gruppe der Glyceride (b-1) eingesetzt wird.

Die erfindungsgemäße Formulierung kann 0,05 bis 10 Gew. % Antioxidantien enthalten. Bevorzugt enthält sie 0,1 bis 6 Gew. % Antioxidantien bezogen auf die zu adsorbierende Komponenten (A).

Die erfindungsgemäße Formulierung kann auch Bindemittel enthalten. Als Beispiele für geeignete Bindemittel sind zu nennen: Lösungen von Kohlehydraten, wie z. B. Glucose, Saccharose, Dextrine und dergleichen, Zuckeralkohole, wie z. B. Mannit, oder Polymerlösungen, wie beispielsweise Lösungen von Hydroxypropylmethylcellulose (HPMC), Polyvinylpyrrolidon (PVP), ethoxilierte Cellulose (EC), Ethylcellulose oder Propylcellulose. Der Bindemittelanteil, bezogen auf das Trockengewicht der pulverförmigen Formulierung kann beispielsweise im Bereich von etwa 0 bis 20 Gew.-%, wie z. B. 1 bis 6 Gew.%, bezogen auf die zu adsorbierende Komponenten (A) je nach Art und Klebeeigenschaften des verwendeten Bindemittels liegen. Zum Binden kann im einfachsten Fall auch Wärme eingesetzt werden.

### Herstellung der Adsorbate

Die zu adsorbierende Komponente (A) wird mit einem Stabilisator (B) gemischt und anschließend auf den Träger (C) aufgebracht. Ebenso möglich ist das Vorlegen des Trägers (C) und die Zugabe der Komponenten (A) und (B) vorgemischt.

### Mischen von (A) und (B)

Die Mischung von (A) und (B) kann nach üblichen Methoden in Abhängigkeit des physikalischen Zustands von (A) und (B) erfolgen. Sowohl (A) als auch (B) können fest oder flüssig vorliegen. Sie können in geeigneten Ölen oder in Wasser oder anderen Lösungsmitteln gelöst oder dispergiert sein.

Im einfachsten Fall erfolgt eine Mischung von 2 Flüssigkeiten, denkbar ist ebenso, dass der Stabilisator (B) aufgeschmolzen wird, die zu adsorbierende Komponenten (A) darin gelöst wird und die Mischung nach Adsorption auf Träger (C) unter Abkühlen erstarrt. Ebenso denkbar ist das Aufschmelzen von (A) und (B), getrennt oder gemeinsam, das homogene Mischen der Schmelzen, die Zugabe der Mischung zum Träger (C) und das anschließende Erstarren. Besonders bevorzugt sind Mischmethoden, die zu einer gleichmäßigen Verteilung von (A) in (B) führen, dazu zählen homogene Schmelzen, Lösungen aber auch Dispersionen.

Dem Fachmann sind die hierzu benötigten Geräte bekannt, z.B. beheizbare Apparate, die bevorzugt mit Mischwerkzeugen, z.B. Rührern versehen sind. Der erforderliche Energieeintrag kann beispielsweise über beheizbare Behälterwände, beheizbare Mischwerkzeuge und/oder mechanischen Energieeintrag erfolgen.

Auch ist es möglich, eine bereits flüssige Komponente so weit zu erwärmten, dass nach Zugeben der zweiten noch nicht geschmolzenen Komponente in den gewünschten Konzentration beide Stoffe danach in flüssigem Zustand vorliegen.

Es ist auch denkbar, den noch festen Stoff durch Eintrag von mechanischer Energie zunächst in der flüssigen Komponente zu dispergieren und beide zusammen in oben genannten Apparaten weiter zu erwärmen.

Liegt (B) als Feststoff mit unterschiedlichsten Partikelgrößenverteilungen oder als (wässrige) Lösungen oder Dispersionen alleine oder mit anderen Stoffen zusammen vor, ist es vorteilhaft (B), mit oder ohne Wasser in den gewünschten Konzentrationen zu (A) zu geben und daraus eine Dispersion herzustellen.

Liegt (B) als pulverförmiger Feststoff vor, und liegt (A) in Form einer Flüssigkeit vor, ist es vorteilhaft, (B) in (A) einzurühren und eine gewünschte Verfestigung beispielsweise durch Temperaturabsenkung der Mischung zu erzielen.

In einer weiteren Ausführungsform kann (A) in einem wässrigen Schutzkolloid (z.B. Gelatine) / Zucker-Phase unter Zusatz von (B) und ggfls. anderen Zuschlagstoffen dispergiert werden. Die Verfestigung kann hier wieder durch Temperaturabsenkung und/oder durch Wasserentzug erfolgen.

Denkbar ist weiterhin durch z.B. Direkteinleitung einer dritten Komponente, z.B. Dampf oder erhitztem Wasser oder anderen Medien, (A) oder (B) oder Mischungen aus (A) und (B) aufzuschmelzen.

Die Vermischung von (A) und (B) kann (gleichzeitig oder nachgeschaltet zur Erwärmung) schonend oder durch intensiven Energieeintrag erfolgen.

In einigen Fällen erfolgt eine ausreichende Vermischung allein infolge von Molekularbewegung und Dichteunterschieden nach ausreichend langer Zeit oder durch langsames Rühren oder Unterheben. Falls erforderlich wird intensives Rühren mit optimierter Geometrie und schnelllaufenden Rühr- oder Mischorganen angewendet.

In einigen Fällen ist eine Drehbewegung oder Schüttelbewegung der kompletten Apparate an sich ausreichend. In anderen Fällen wird der notwendige Energieeintrag zur Vermischung durch Pumpen der Stoffe durch statische Mischer, Blenden oder auch andere Dispergiermaschinen erfolgen. Der Energieeintrag kann auch z.B. über Ultraschallsonotroden erfolgen.

### Adsorbatherstellung: Aufbringen von (A) in Mischung mit (B) auf (C) im Mischer

Es können diskontinuierlich oder kontinuierlich arbeitende Mischer eingesetzt werden. Der Träger (C) wird ggfls. zusammen mit Zuschlagstoffen vorgelegt. Pflugscharen, Schaufeln, Schnecken oder ähnliches sorgen für eine mehr oder minder intensive Produktdurchmischung. Klassische Beispiele sind Pflugscharmischer, Konusschneckenmischer oder ähnliche Apparate.

Eine Beschreibung von Mischverfahren und Mischern findet sich z.B. in einer technischen Information von Degussa(-Hüls), TI 1213, "Kieselsäuren als Fließhilfsmittel und als Trägersubstanz - Geeignete Mischverfahren für Pulver und Granulate".

Auch sehr flache, kasten- oder trogförmige Bauformen mit einer oder mehreren Schnecken sind einsetzbar. Weitere Bauformen sind schnelllaufende Mischer wie z.B. der Turbolizer ® Mixer/Coater von Hosokawa Micron B.V.

Vorzugsweise können diskontinuierlich arbeitende Mischer eingesetzt werden. Das Trägermaterial wird ggf. zusammen mit Zuschlagstoffen vorgelegt. Pflugscharen, Schaufeln, Schnecken oder ähnliches sorgen für eine mehr oder minder intensive Produktdurchmischung. Klassische Beispiele sind Pflugscharmischer, Konusschneckenmischer oder ähnliche Apparate. Alternativ ist die Produktdurchmischung über eine Bewegung des gesamten Behälters möglich. Beispiele hierfür sind Taumelmischer, Trommelmischer oder ähnliches. Eine weitere Möglichkeit besteht in der Verwendung von pneumatischen Mischern (siehe Ullmann's Encyclopedia of Industrial Chemistry, Sixth Edition, Mixing of Solids).

Die Dosierung/Zugabe der zu adsorbierenden Komponente (A) in Mischung mit Stabilisator (B) vorliegend, erfolgt gegebenenfalls zusammen mit weiteren Stoffen (E) i.d.R. über Einrichtungen zum Auftropfen oder Aufdüsen. Beispiele hierfür sind Lanzen, Brauseköpfe, Einstoff- oder Mehrstoffdüsen, in seltenen Fällen rotierende Tropf- oder Zerstäubungseinrichtungen. Im einfachsten Fall ist die Zugabe auch lokal als konzentrierter Strahl möglich. Alternativ kann im Mischer zunächst die zu adsorbierenden Komponente (A) in Mischung mit Stabilisator (B) vorliegend, vorgelegt werden, um danach den Träger (C) aufzugeben.

Die Zugabe der zu adsorbierenden Komponente (A), in Mischung mit Stabilisator (B) vorliegend, kann bei Überdruck, Normaldruck oder bei Unterdruck gegen Atmosphäre, vorzugsweise bei Normaldruck und Unterdruck erfolgen.

In einzelnen Fällen kann es vorteilhaft sein, die zu adsorbierende Komponente (A) vorzuheizen (Erniedrigung der Viskosität, Veränderung der Benetzungseigenschaften), sowie Wärme über die Behälterwand und/oder die Mischwerkzeuge zuzuführen oder zu entziehen. In einzelnen Fällen kann es erforderlich sein, Wasser- oder Lösungsmitteldämpfe abzuführen.

In Abhängigkeit von der zu adsorbierende Komponente (A) wählt der Fachmann den oder die Stabilisatoren vorzugsweise so, dass eine homogene Mischung aus (A) und (B) hergestellt werden kann, diese kann beispielsweise auch in Form einer Dispersion vorliegen.

Die Herstellung der Adsorbate erfolgt in der Regel bei Temperaturen, die über dem Schmelzpunkt der Mischungen liegt.

(B) wird in der Regel so gewählt, dass die Mischung von (B) mit der zu adsorbierenden Komponenten (A) bei Temperaturen unter 80 °C, bevorzugt unter 60 °C, insbesondere unter 40 °C, fest und homogen vorliegen.
Zur Erhöhung der Beladung des Trägers (C) und zur Minimierung von Sauerstoffeinschlüssen kann es günstig sein, den den Träger enthaltenden Mischer vor Zugabe des Wirkstoffs zu evakuieren sowie ggfls. mit Schutzgas zu überdecken. In Abhängigkeit vom Träger ist dies mehrfach zu wiederholen.

Alternativ sind kontinuierlich arbeitende Mischer geeignet. Die Zugabe von zu adsorbierenden Komponenten (A) in Mischung mit (B), und den gegebenenfalls weiteren Stoffe (E) und Träger (C) erfolgt dabei vorzugsweise an unterschiedlichen Orten im Mischer.

Die Herstellung von Adsorbaten kann diskontinuierlich oder kontinuierlich in Wirbelschichten erfolgen. Die Bewegung der Träger (C) erfolgt durch das ggf. heiße Wirbelgas. Als Wirbelgas ist Luft oder auch Inertgas geeignet. In Einzelfällen ist es sinnvoll, über die Behälterwand und/oder über in die Wirbelschicht eingetauchte Wärmetauscherflächen, Wärme zuzuführen oder zu entziehen. Geeignete Wirbelschichten sowie die erforderliche Peripherie sind Stand der Technik.

Die diskontinuierliche oder kontinuierliche Dosierung und ggf. die Vorheizung der zu adsorbierenden Komponenten (A) in Mischung mit (B), erfolgen durch die oben beschriebenen Einrichtungen, die dem Fachmann bekannt sind.

Die Herstellung von Adsorbaten kann in Einzelfällen vorteilhaft durch Kombination von Mischer und Wirbelschicht erfolgen.

Enthalten die Adsorbate weitere Stoffe (E) so können diese zusammen Komponenten (A) zugegeben werden und/oder mit dem Träger (C) gemischt werden.

In Einzelfällen kann es vorteilhaft sein, bei der Herstellung Adsorbate im Mischer oder unmittelbar danach/davor Puderungsmittel wie Talkum, Silikate oder ähnliches zum Vermeiden von Verklebungen zuzugeben.

Besonders bevorzugt ist es die Mischung aus (A) und (B) z.B. durch Temperaturabsenkung in oder teilweise auf (C) zu fixieren. Es ist von Vorteil durch Wahl der geeigneten Temperaturen die Viskosität der Mischung aus (A) und (B) soweit zu erniedrigen, dass eine ausreichend schnelle und ausreichend vollständige Beladung des Trägers (C) erfolgen kann. Besonders vorteilhaft ist dieses Verfahren, falls (B) mindestens eine Verbindung aus (b-1) und mindestens eine Verbindung aus (b-2) enthält.

Die Aufheizung der Mischung aus (A) und (B) kann beispielsweise in vorgeschalteten separaten Behältern oder Apparaten oder beheizbaren Rohrleitungen erfolgen. Die Träger (C) können falls erforderlich ebenfalls vorgeheizt zugegeben werden. Die Aufheizung der Mischung aus (A) und (B) sowie von (C) kann zusammen oder getrennt auch im Mischer selbst erfolgen. Die Aufheizung kann erfolgen durch Wärmetausch über die Behälterwand oder beheizter Mischorgane oder über den Eintrag von mechanischer Rühr- oder Mischenergie.

Die Abkühlung der Mischung kann wiederum im Mischer selbst durch Wärmetausch über die Behälterwand oder kühlbarer Mischorgane oder in seltenen Fällen durch Ausnutzung von Verdunstungs- bzw. Verdampfungskühlung erfolgen. Auch eine Kühlung in nachgeschalteten Apparaten oder im einfachsten Fall durch Wärmeaustausch mit der Umgebung beim Lagern ist selbstverständlich möglich.

Werden als Stabilisatoren (B) Verbindungen aus der Stoffklasse der Emulgatoren (b-2) eingesetzt hat es sich als vorteilhaft erwiesen zur Herstellung der Mischung von (A) und (b-2), sowie gegebenenfalls weiterer Stabilisatoren, folgendes Verfahren anzuwenden:
(a) Herstellen einer Mischung aus Emulgator (b-2) und (A) und gegebenenfalls Wasser und Zugabe dieser Mischung auf den im Mischer vorgelegten Träger (C)

Soweit erforderlich werden die einzelne Komponenten, (A), Mischungen aus (A) und (B) bei Bedarf aufgeheizt und gekühlt.

Die genannten Verfahren haben sich auch als besonders vorteilhaft erwiesen, falls der Stabilisator (B) ausgewählt ist aus der Gruppe der Polysaccharide (b-3).

In einzelnen Fällen kann es bei der Herstellung der Adsorbate erforderlich sein, Wasser- oder Lösungsmitteldämpfe abzuführen. Dies kann im Mischer selbst über zugehörige Filter erfolgen oder in nachgeschalteten Apparaten wie z.B. in weiteren Trocknern, Mischern, Rührbehältern, Wirbelschichten, Sprühtrocknem, Prilltürmen, etc. vorzugsweise bei Unterdruck oder Normaldruck.

In einer weiteren Ausführungsform der Erfindung werden die Adsorbate nach ihrer Herstellung beschichtet. Dies kann direkt im Mischer oder in nachgeschalteten Apparaten durchgeführt werden. Dies kann durch Zugabe von flüssigem Beschichtungsmitteln wie unter (D) beschrieben, erfolgen oder durch Zugabe von zunächst festem Beschichtungsmaterial (D), das infolge einer Wandbeheizung des Apparats oder der Welle oder infolge des mechanischen Energieeintrags schmilzt oder erweicht und das Adsorbat überzieht.

In einer weiteren Ausführungsform können das Adsorbat und das vorzugsweise pulverförmige Beschichtungsmaterial (D) im Mischer vorgelegt werden, gegebenenfalls vorgemischt werden und es kommt so infolge hohem mechanischem Energieeintrags im selben oder in separaten Apparaten (Beispiele sind alle bereits genannten Mischer aber auch langsamlaufende Mühlen und Trockner) zur Beschichtung des Adsorbates.

In einzelnen Fällen kann es vorteilhaft sein Adsorbat und/oder Beschichtungsmittel (D) vorzuheizen oder zu kühlen (Veränderung von Viskosität, Veränderung der Benetzungseigenschaften, Beeinflussung der Erstarrungseigenschaften) sowie Wärme über die Behälterwand und/oder die Mischwerkzeuge zuzuführen oder zu entziehen. In einzelnen Fällen kann es erforderlich sein Wasser- oder Lösungsmitteldämpfe abzuführen. Eine Veränderung der Benetzungseigenschaften kann auch durch Zugabe oberflächenaktiver Substanzen erreicht werden.

Das Aufbringen von Beschichtungsmaterialien dient dem zusätzlichen Schutz des Wirkstoffs, der Verzögerung oder Beschleunigung der Wirkstofffreisetzung, der Verstärkung des Wirkmechanismus oder der Erzielung additiver Effekte.

Die Erfindung schließt mit ein, dass beim Aufbringen der Beschichtungsmittel oder unmittelbar danach/davor Puderungsmittel wie Talkum, Silikate oder ähnliches zum Vermeiden von Verklebungen zuzugeben werden kann.

### Adsorbatherstellung: Aufbringen von (A) in Mischung mit (B) auf (C) in der Wirbelschicht

In einer weiteren Ausführungsform der Erfindung kann die Herstellung der Adsorbate diskontinuierlich oder kontinuierlich in Wirbelschichten erfolgen. Die Bewegung der Partikeln erfolgt durch das gegebenenfalls heiße oder gekühlte Wirbelgas. Als Wirbelgas sind z.B. Luft oder auch Inertgas (z.B. Stickstoff) geeignet. In Einzelfällen ist es sinnvoll, über die Behälterwand sowie über in die Wirbelschicht eingetauchte Wärmetauscherflächen Wärme zuzuführen oder zu entziehen. Geeignete Wirbelschichten sowie die erforderliche Peripherie sind im Stand der Technik beschrieben.

Die diskontinuierliche oder kontinuierliche Dosierung und ggfls. die Vorheizung von (A), vorzugsweise von Mischungen von (A) und (B) und gegebenenfalls weiteren Stoffe (E) sowie von Zuschlagstoffen erfolgt analog den bei der Mischung beschriebenen Vorgehensweisen.

Beispielsweise kann der Träger (C) in einem Wirbelbett vorgelegt werden. Dieser wird verwirbelt und durch Aufsprühen, Auftropfen, etc. einer wässrigen oder nichtwässrigen Lösung oder Dispersion oder einer Schmelze von einer Mischung von (A) und (B) mit diesen in der gewünschten Konzentration beladen.

Hilfreich sind nach dem Stand der Technik bekannte Einbauten, welche eine gezielte Durchmischung des zu beladenden Trägers (C) unterstützen. Beispiele hierfür sind drehende Verdrängungskörper, Wursterrohre aber auch speziell gefertigte Wirbelbodengeometrien (Neigung und/oder Perforierung des Bodens) oder die Unterstützung der gezielten Feststoffbewegung durch sinnvoll angeordnete Düsen, z.B. tangential angeordnete Einstoff- oder Zweistoff oder Mehrstoffdüsen.

In einer weiteren Ausführungsform der Erfindung kann die Herstellung der erfindungsgemäßen Adsorbate durch Kombination von Mischer und Wirbelschicht erfolgen.

Selbstverständlich ist auch in Wirbelschichten das Aufbringen einer nachträglichen Beschichtung möglich.

### Adsorbate und ihre Verwendung

Die Erfindung umfasst weiterhin Adsorbate, welche nach den vorgenannten Verfahren erhältlich sind. Diese Adsorbate zeichnen sich durch eine erhöhte Stabilität der zu adsorbierenden Komponenten (A) aus und sind gleichzeitig großtechnisch einfach herstellbar. Sie lassen sich deshalb in einer Vielzahl von lebensmitteltechnischen Anwendungen, kosmetischen Zubereitungen sowie Tierfutterzubereitungen einsetzen. Sie sind insbesondere in komplexen Matrices, wie beispielsweise Prämixen der Tierfutterindustrie einsetzbar.

Die erfindungsgemäßen Adsorbate weisen in der Regel unbeschichtet eine Beladung [definiert als Gew.% der zu adsorbierende Komponenten (A) bezogen auf Gew.-% Träger (C)] von 0,01 bis 10,0, insbesondere 0,1 bis 5,0, bevorzugt 0,5 bis 2,5 auf.

Die erfindungsgemäßen Adsorbate weisen in der Regel unbeschichtet eine Gesamtbeladung [definiert als Summe der Gew.-% der zu adsorbierenden Komponenten (A) und Stabilisatoren (B) bezogen auf Gew.% Träger (C)] von 0,02 bis 15,0; insbesondere 0,05 bis 10, insbesondere 0,2 bis 8,0; bevorzugt 0,2 bis 5,0, besonders bevorzugt 0,5 bis 2,5 auf.

### BEISPIELE

Alle Angaben in Gew.-%

**Verwendete Abkürzungen und Handelsprodukte**

| | |
|---|---|
| EQ | Ethoxyquin |
| CLA-ME | konjugierte Linolsäure Methylester |
| Sipemat 22^{®} | Kieselsäure der Fa. Degussa |
| Tixosil 38 X^{®} | Kieselsäure der Fa. Rhodia |
| N-Zorbit M | Maltodextrin auf Basis Tapioka der Fa. National Starch |

### Vergleichsbeispiele

### V 1a: Beladen von Trägerkieselsäure mit CLA in der Wirbelschicht

Für die Versuchsdurchführung stand ein Laborwirbelbett der Fa. Niro- Aeromatic, Typ MP-1, zur Verfügung. Als Vorlagegefäß wurde ein Kunststoffkonus mit einem Anströmboden-Durchmesser von 110 mm und ein Lochboden mit 8 % freier Fläche eingesetzt.

Als Trägermaterial wurde Tixosil 38 X, eine Kieselsäure von Rhodia, eingesetzt. 323 g dieser Kieselsäure wurden im Becherglas auf 70 °C im Trockenschrank erwärmt und danach als Vorlagematerial in den Konus des Laborwirbelbetts eingefüllt.
Die im Wirbelbett vorgelegte Kieselsäure wurde bei einer Luftmenge von 10 bis 15 m³/h und einer Zulufttemperatur von 45 bis 50 °C gleichmäßig fluidisiert. Der CLA-Methylester (396 g) wurde bei 1 bar Sprühdruck durch Unterdruckeinsaugung im Topsprayverfahren mit einer 1,0 mm Zweistoffdüse auf die vorgelegte Kieselsäure aufgesprüht. Die Sprühdauer betrug ca. 26 min.
Man erhielt gut fließfähige Adsorbate mit einem Trocknungsverlust von ca. 2%.

### V 1b: Beschichtung des in V1a erhaltenen Adsorbats

Das aus V1a erhältlich Adsorbat wurde in einem zweiten Arbeitsschritt in derselben Laborwirbelschicht mit einer wässrigen Gelatine-Zucker-Dispersion überzogen. Anschließend wurde Glutardialdehyd zur Vernetzung aufgesprüht.

### Vergleichsbeispiel V2a und V 2b Herstellung von Adsorbaten

### Beispiel V 2a

In einem Pflugscharmischer (Loedige Typ M5 GR) wurden 802 g eines Kieselsäure-Trägerstoffs (Sipernat 22^{®} , Degussa, Trocknungsverlust 5,6%) vorgelegt. Unter Rühren bei ca. 200 U/min wurde ein Gemisch aus 810 g CLA-Methylester-Öl und 8,0 g Ethoxyquin über eine Sprühpistole innerhalb von 4 min aufgesprüht. Anschließend wurde noch 5 min bei einer Rührgeschwindigkeit von 345 U/min nachgerührt. Man erhielt ein gut fließfähiges Pulver, Zusammensetzung siehe Tabelle.

### Beispiel V 2b

Im gleichen Mischer wurden unter Zugabe von CLA-Methylester-Öl auf einem Kieselsäureträger (Tixosil 38 X^{®} ) in gleicher Vorgehensweise gut fließfähige Pulver hergestellt.

| Beispiel | Beladung | Träger (C) Kieselsäure | Rezeptur in % | | | |
|---|---|---|---|---|---|---|
| | | | Kieselsäure | (A) CLA-ME | (E) EQ | Restfeuchte |
| V2a | 1,07 | Sipemat 22 | 46,7 | 50,0 | 0,5 | 2,8 |
| V2b | 1,18 | Tixosil 38X | 44,1 | 52,0 | 0,5 | 3,4 |

### Retentionen der Adsorbate im Prämixtest (Ferkelfutter)

Die Retentionen wurden bei folgenden Versuchsbedingungen untersucht: Lagertemperatur 40 °C, rel. Luftfeuchte 70 %, Lagerort Klimaschrank, Packmittel 25 ml Penicillingläser, offen. Einwaage: ca. 25 mg reinem CLA-Methylester bzw. CLA-Ethylester entsprechende Menge an Adsorbat. Ferkelfutter (Mehl) 5 g. Analysenzeitpunkte Ausgangsbestimmung, 2 und 4 Wochenwert (HPLC, jeweils 2-fach-Bestimmung).

Durchführung:CLA-Methylester bzw. CLA-Ethylester enthaltenden Adsorbate werden mit dem Ferkelfutter gemischt und bei einer Temperatur von 40 °C und einer relativen Feuchte von 70 % über einen Zeitraum von 4 Wochen offen in einem Klimaschrank gelagert. Nach 2 bzw. 4 Wochen werden die Retentionen bezogen auf den jeweiligen Ausgangswert ermittelt.

Beurteilung der Proben (2 Wochen bei 40 °C/70 % r.F.):
Retention > 70 % sehr gut; Retention 50-70 % gut, Retention 30-50 % annehmbar

Die Retention ist definiert als Gehalt an Wirkstoff nach 14 Tagen im Vergleich zum eingesetzten Gehalt an Wirkstoff.

Die Stabilität des CLA-ME (Komponenten (A)) in den Adsorbaten hergestellt gemäß Vergleichsbeispielen V2a und V2b wurde im Prämixtest bestimmt. Der Prämixtest ergab nach 14 Tagen folgende Retentionen:

| Beispiel | Retention |
|---|---|
| V2a | 36 % |
| V2b | 33 % |

### Erfindungsgemäße Beispiele

### Beispiele 1a bis 1 g

### Herstellung stabiler Adsorbate unter Verwendung von Stabilisatoren (B) aus der Stoffklasse der Glyceride (b-1)

Zunächst wurden die ausgewählten Glyceride eingewogen und auf eine Temperatur, die um 20 °C über der jeweiligen Schmelztemperatur des Glycerids liegt, erwärmt und dabei vollständig aufgeschmolzen. Die gewünschte Menge an Komponenten (A), hier CLA-Methylester-Öl, stabilisiert mit 1 % Ethoxyquin (Komponente E), wurde parallel auf dieselbe Temperatur erwärmt. Beide Flüssigkeiten wurden danach in ein Becherglas zusammengeschüttet und mit dem U-Turrax zwei Minuten bei 5000 U/min dispergiert.

Parallel wurde in einen Pflugscharmischer (Loedige Typ M5 GR) 640g Tixosil 38 X^{®} , Rhodia, vorgelegt.

Unter Rühren bei ca. 200 U/min wurde nun das vorbereitete Gemisch aus CLA-Methylester-Öl, Ethoxyquin und Glycerid mit Hilfe einer Sprühpistole aufgesprüht. Anschließend wurde noch 5 min bei einer Rührgeschwindigkeit von 345 U/min nachgerührt und danach die erhaltenen stabilen Adsorbate in eine Schüssel abgefüllt. Man erhielt jeweils Pulver mit folgenden Zusammensetzungen:

| **Nr.** | **Beladung / Gesamtbeladung** | **(B) Glycerid** | **Rezeptur in %** | | | |
|---|---|---|---|---|---|---|
| | | | **(C) Kieselsäure Tixosil 38X™** | **(A) CLA-ME incl.1 Gew.% EQ (E)** | **(B) Glycerid** | **Restfeuchte** |
| | | | | | | |
| **1 a** | **0,9/1,4** | Cutina CP | 40,2 | 37,1 | 20,1 | 2,6 |
| **1 b** | **1,2/1,4** | Cutina CP | 40,2 | 47,2 | 10,1 | 2,5 |
| **1 c** | **0,9/1,4** | Precirol ATO 5 | 40,1 | 37,1 | 20,1 | 2,7 |
| **1 d** | **1.2/1,4** | Precirol ATO 5 | 40,2 | 47,3 | 10,1 | 2,4 |
| **1 e** | **0,9/1,4** | Biogapress | 40,1 | 37,1 | 20,1 | 2,7 |
| **1 f** | **1,2/1,4** | Biogapress | 40,2 | 47,2 | 10,1 | 2,5 |
| **1 g** | **0,9/1,4** | Vegeol PR-267 | 40,2 | 37,1 | 20,1 | 2,6 |

Retentionen der Adsorbate gemäß Beispielen 1a bis 1 g im Prämixtest

Der Prämixtest ergab nach 14 Tagen folgende Retentionen:

| Bespiel | Retention |
|---|---|
| 1 a | 69 % |
| 1 b | 64 % |
| 1 c | 61% |
| 1 d | 71% |
| 1 e | 58 % |
| 1 f | 67 % |
| 1 g | 61 % |

### Beispiele 2a und 2b

### Herstellung stabiler Adsorbate unter Verwendung von Stabilisatoren (B) aus der Stoffklasse der Emulgatoren (b-2)

Zunächst wurde in einen Pflugscharmischer (Loedige Typ M5 GR) 640g Tixosil 38 X^{®} , Rhodia, vorgelegt.

Im **Beispiel 2a** wurde die gewünschte Menge an CLA-Methylester-Öl (Komponente A), stabilisiert mit 1 % Ethoxyquin (Komponente E), mit Cremophor CO 40 (Komponente b-2) gemischt und mit dem U-Turrax 30 Sekunden bei 5000 U/min vordispergiert. Das Wasser wurde dazugegeben und diese Mischung mit dem U-Turrax 10 Minuten bei 8800 U/min emulgiert. Diese Mischung wurde in eine Sprühpistole gefüllt und unter Rühren auf die im Mischer vorgelegte Kieselsäure aufgesprüht. Ansonsten wurde wie in Beispiel 1 beschrieben vorgegangen.

Im **Beispiel 2b** wurde die gewünschte Menge an CLA-Methylester-Öl, stabilisiert mit 1 % Ethoxyquin, mit Cremophor CO 40 gemischt und mit dem U-Turrax zwei Minuten bei 10000 U/min dispergiert. In Abwesenheit von zusätzlichem Wasser wurde die Emulgator/CLA/EQ-Mischung mit der Sprühpistole auf die Kieselsäure aufgesprüht. Ansonsten wurde wie in Beispiel 2a beschrieben vorgegangen.

Man erhielt jeweils Adsorbate mit folgenden Zusammensetzungen:

| **Beispiel** | **Beladung/ Gesamtbeladung** | **Rezeptur in %** | | | |
|---|---|---|---|---|---|
| | | **(C) Tixosil 38X** | **(A) CLA-ME incl. EQ** | **(B) Cremophor CO 40** | **Wasser** |
| | | | | | |
| **2a** | **1,19/1,28** | 40,0 | 48,4 | 2,9 | 8,7 |
| **2b** | **1,33/1,43** | 40,0 | 54,1 | 2,9 | 3,0 |

### Retentionen der Adsorbate aus Beispielen 2a und 2b im Prämixtest

Im Prämixtest wurden die oben angeführten und im Lödigemischer hergestellten stabilen Adsorbate getestet. Der Prämixtest ergab nach 14 Tagen folgende Retentionen:

| Beispiel | Retention |
|---|---|
| 2a | 64 % |
| 2b | 80 % |

### Beispiele 3 Herstellung stabiler Adsorbate unter Verwendung von Stabilisatoren (B) aus der Stoffklasse der Polysaccharide (b-3)

Zunächst wurde in einen Pflugscharmischer (Loedige Typ M5 GR) 640g Tixosil 38 X^{®} , Rhodia, vorgelegt. CLA-Methylester-Öl, stabilisiert mit 1 % Ethoxyquin, wurde im Becherglas auf 70 °C erwärmt und die gewünschte Menge N-Zorbit M untergerührt. Diese Mischung wurde in eine Sprühpistole gefüllt und unter Rühren auf die im Mischer vorgelegte Kieselsäure aufgesprüht. Ansonsten wurde wie in Beispiel 1a beschrieben vorgegangen.

Man erhielt ein Pulver mit folgender Zusammensetzung:

| **Beispiel** | **Rezeptur in %** | | | |
|---|---|---|---|---|
| | **(C) Tixosil 38X** | **(A) CLA-ME incl. EQ (E)** | **(B) N-Zorbit M** | **Wasser** |
| **3** | 40,0 | 48,2 | 8,8 | 3,0 |

Retentionen der Adsorbate nach Beispiel 3 im Prämixtest (nach 14 Tagen)

| Beispiel | Beladung/Gesamtbeladung | Retention |
|---|---|---|
| 3 | 1,18 / 1,43 | 67 % |

### Beispiel 4a bis 4c: Herstellung von Adsorbaten unter Verwendung von Stabilisatoren (B) aus der Stoffklasse der Chelatoren (b-4)

CLA-Methylester-Öl, stabilisiert mit 1 % Ethoxyquin, wurde in einem ersten Schritt mit verschiedenen Chelatoren (b-4) vermischt. Soweit erforderlich wurde Wasser und zusätzlich als Stabilisator der Emulgator (b-1) Cremophor CO 40 zugegeben.

Mit Hilfe dieser Lösungen bzw. Dispersionen wurden analog zu Beispiel 1a wiederum Adsorbate im Lödigemischer mit folgender Zusammensetzung hergestellt.

| **Beispiel** | **Rezeptur in %** | | | |
|---|---|---|---|---|
| | **(C)Tixosil 38X** | **(A) CLA-ME incl. EQ (E)** | **Chelator (B)** | **Wasser** |
| | | | | |
| **4a** | 39,9 | 54,1 | 2,9 % Salicylsäure | 3,1 |
| **4b** | 40,1 | 54,2 | 2,9 % Stearylcitrat | 2,8 |
| **4c** | 40,2 | 49,4 | 2,5 % Weinsäure + 2,5 % Cremophor CO 40 | 5,4 |

### Retentionen der Adsorbate 4a bis 4b im Prämixtest

Der Prämixtest ergab nach 14 Tagen folgende Retentionen:

| Beispiel | Beladung / Gesamtbeladung | Retention |
|---|---|---|
| **4a** | 1,33 / 1,43 | 70 % |
| **4b** | 1,32 / 1,42 | 69 % |
| **4c** | 1,2 / 1,36 | 62 % |

## Patentansprüche

1. Verfahren zur Herstellung von für die menschliche oder tierische Ernährung sowie kosmetische Anwendungen geeigneten Adsorbaten bei dem man eine zu adsorbierenden Komponente (A), auf einen Träger (C) unter Einsatz mindestens eines Stabilisators (B) aufbringt, wobei die mittlere Partikelgröße des Trägers (C) mindestens 80 µm beträgt,
wobei der Stabilisator (B) ausgewählt ist aus der Gruppe bestehend aus Glyceriden (b-1),
Emulgatoren (b-2) ausgewählt aus
nichtionogenen Tensiden aus mindestens einer der folgenden Gruppen
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
(2) C12/18-Fettsäuremono- und -diester von Anlagerungsprodukten von 1 bis 30 Mol Ethylenoxid an Glycerin,
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte,
(4) Alkylmono- und -oligoglycoside mit 8 bis 22 Kohlenstoffatomen im Alkylrest und deren ethoxylierte Analoga,
(5) Anlagerungsprodukte von 15 bis 60 Mol Ethylenoxid an Ricinusöl und/oder gehärtetes Ricinusöl,
(6) Polyol- und Polyglycerinester,
(7) Anlagerungsprodukte von 2 bis 15 Mol Ethylenoxid an Ricinusöl und/oder gehärtetes Ricinusöl,
(8) Partialester auf Basis linearer, verzweigter, ungesättigter bzw. gesättigter C6/22-Fettsäuren, Ricinolsäure sowie 12-Hydroxystearinsäure und Glycerin, Polyglycerin, Pentaerythrit, Dipentaerythrit, Zuckeralkohole, Alkylglucoside sowie Polyglucoside,
(9) Mono-, Di- und Trialkylphosphate sowie Mono-, Di- und/oder Tri-PEG-alkylphosphate und deren Salze,
(10) Wollwachsalkohole,
(11) Polysiloxan-Polyalkyl-Polyether-Copolymere bzw. entsprechende Derivate,
(12) Mischester von Fettsäuren mit 6 bis 22 Kohlenstoffatomen, Methylglycose und Polyolen,
(13) Polyalkylenglycole
zwitterionischen Tensiden,
ampholytischen Tenside,
quartäre Emulgatoren,
Glycerophospholipiden und Glyceroglycolipiden,
Polysacchariden (b-3) und/oder
Chelatoren (b-4), und wobei
der Träger (C) Kieselsäure ist, und wobei
man die zu adsorbierende Komponente (A) mit einem Stabilisator (B) mischt und anschließend auf einen Träger (C) aufbringt, **dadurch gekennzeichnet, dass**
die zu adsorbierende Komponente (A) konjugierte Octadecapolyensäuren sind, bei denen weniger als 5% des Fettsäureanteils 11,13-Octadecadiensäure-Isomere, 8,10-Octadecadiensäure-Isomere, cis-,cis-Octadecadiensäure-Isomere oder trans-, trans-Octadecadiensäure-Isomere oder Gemische dieser Isomeren sind.

2. Verfahren nach Anspruch 1, **dadurch gekennzeichnet, dass** die mittlere Partikelgröße des Trägers (C) mindestens 100 µm beträgt, bevorzugt mindestens 200 µm beträgt.

3. Verfahren nach einem der vorhergehenden Ansprüche, **dadurch gekennzeichnet, dass** die Menge an Träger (C) größer gleich 10 Gew.% bezogen auf das Adsorbat beträgt.

4. Verfahren nach einem der vorhergehenden Ansprüche, **dadurch gekennzeichnet, dass** das Verhältnis von Stabilisator (B) zu adsorbierender Komponenten (A) kleiner gleich 10, insbesondere kleiner gleich 5, insbesondere kleiner gleich 1, bevorzugt kleiner gleich 0,8 ist

5. Verfahren nach einem der vorhergehenden Ansprüche, **dadurch gekennzeichnet, dass** als Stabilisator (B) mindestens eine Verbindung aus der Gruppe der Glyceride (b-1) und mindestens eine Verbindung aus der Gruppe der Emulgatoren (b-2) ausgewählt wird.

6. Verfahren nach einem der vorhergehenden Ansprüche, **dadurch gekennzeichnet, dass** man das Adsorbat beschichtet.

7. Verfahren nach Anspruch 6, **dadurch gekennzeichnet, dass** das Beschichtungsmittel (D) ausgewählt ist aus der Gruppe bestehend aus
a) Polyalkylenglycole, insbesondere Polyethylenglycolen mit einem zahlenmittleren Molekulargewicht von etwa 400 bis 15000,
b) Polyalkylenoxid-Polymeren oder -Copolymeren mit einem zahlenmäßigen Molekulargewicht von etwa 4000 bis 20000, insbesondere Blockcopolymere von Polyoxyethylen und Polyoxypropylen;
c) Substituierte Polystyrole, Maleinsäurederivate und Styrolmaleinsäurecopolymere;
d) Polyvinylpyrrolidone mit einem zahlenmittleren Molekulargewicht von etwas 7000 bis 1000000;
e) Vinylpyrrolidon/Vinylacetat-Copolymere mit einem zahlenmittleren Molekulargewicht von etwa 30 000 bis 100 000;
f) Polyvinylalkohol mit einem zahlenmittleren Molekulargewicht von etwa 10 000 bis 200000, Polyphthalsäurevinylester;
g) Hydroxypropylmethylcellulose mit einem zahlenmittleren Molekulargewicht von etwa 6000 bis 80 000;
h) Alkyl(meth)acrylat-Polymere und -Copolymere mit einem zahlenmittleren Molekulargewicht von etwa 100 000 bis 1 000 000, insbesondere Ethylacrylat/Methylmethacrylat-Copolymere und Methacrylat/Ethylacrylat-Copolymere;
i) Polyvinylacetat mit einem zahlenmittleren Molekulargewicht von etwa 250000 bis 700000 ggfls. stabilisiert mit Polyvinylpyrrolidon;
j) Polyalkylenen, insbesondere Polyethylenen;
k) Phenoxyessigsäure-Formaldehyd-Harz;
l) Cellulosederivate, wie Ethylcellulose, Ethylmethylcellulose, Methylcellulose, Hydroxypropylcellulose, Hydroxypropylmethyl-cellulose, Carboxymethylcellulose, Celluloseacetatphthalat;
m) Tierische, pflanzliche oder synthetische Glyceride;
n) Tierische, pflanzliche oder synthetische Wachse oder chemisch modifizierte tierische, pflanzliche Wachse wie Bienenwachs, Candelillawachs, Carnaubawachs, Montanesterwachs und Reiskeimölwachs, Walrat, Lanolin, Jojobawachs, Sasolwachs, Japanwachs oder Japanwachsersatz;
o) Tierische und pflanzliche Proteine wie z.B. Gelatine, Gelatinederivate, Gelatineersatzstoffe, Casein, Molke, Keratin, Sojaprotein; Zein und Weizenprotein;
p) Mono- und Disaccharide, Oligosaccharide, Polysaccharide, z.B. Stärken, modifizierte Stärken sowie Pektine, Alginate, Chitosan, Carrageene;
q) pflanzliche Öle, z.B. Sonnenblumen-, Distel-, Baumwollsaat-, Soja-, Maiskeim-, Oliven-, Raps(samen)-, Lein-, Ölbaum-, Kokos-, (Öl)Palmkernöl und Palmöl;
r) synthetische oder halbsynthetische Öle, z.B. mittelkettige Triglyceride oder Mineralöle;
s) Tierische Öle wie z.B. Hering-, Sardine- und Walöl;
t) gehärtete (hydrierte oder teilhydrierte) Öle/Glyceride wie z.B. von den oben genannten, insbesondere hydriertes Palmöl, hydriertes Baumwollsaatöl, hydriertes Sojaöl;
u) Lackcoatings wie z.B. Terpene, insbesondere Schellack, Tolubalsam, Perubalsam, Sandarak, und Silikonharze;
v) Fettsäuren, sowohl gesättigte als auch einfach und mehrfach ungesättigte C6 bis C24-Carbonsäuren;
w) Kieselsäuren.

8. Verfahren nach einem der vorhergehenden Ansprüche, **dadurch gekennzeichnet, dass** das Adsorbat weitere Stoffe (E) enthält, ausgewählt aus der Gruppe bestehend aus Antioxidantien, Konservierungsmittel, Bindemittel und / oder Geschmacksstoffe.

9. Verfahren nach Anspruch 8, **dadurch gekennzeichnet, dass** die weiteren Stoffe (E) zusammen mit Komponenten (A) zugegeben werden.

10. Verfahren nach Anspruch 8, **dadurch gekennzeichnet, dass** die weiteren Stoffe (E) mit dem Träger gemischt werden.

11. Adsorbate, erhältlich nach einem der vorgenannten Verfahren.

12. Verwendung der Adsorbate nach Anspruch 11 in Lebensmitteln, in kosmetischen Zubereitungen, in Zubereitungen der Tierernährung, insbesondere in Prämixen.

## Claims

1. A process for producing adsorbates suitable for human or animal nutrition and also cosmetics applications in which a component (A) that is to be adsorbed is applied to a carrier (C) using at least one stabilizer (B), the mean particle size of the carrier (C) being at least 80 µm,
the stabilizer (B) being selected from the group consisting of
glycerides (b-1)
emulsifiers (b-2) selected from
non-ionogenic surfactants from at least one of the following groups
(1) Addition products of from 2 to 30 mol of ethylene oxide and/or from 0 to 5 mol of propylene oxide to linear fatty alcohols containing from 8 to 22 carbons, to fatty acids containing from 12 to 22 carbons and to alkylphenols containing from 8 to 15 carbons in the alkyl group,
(2) C_{12/18}-fatty acid monoesters and diesters of addition products of from 1 to 30 mol of ethylene oxide to glycerol,
(3) Glycerol monoesters and diesters and sorbitan monoesters and diesters of saturated and unsaturated fatty acids containing from 6 to 22 carbons and their ethylene oxide addition products,
(4) Alkyl monoglycosides and oligoglycosides containing from 8 to 22 carbons in the alkyl radical and ethoxylated analogs thereof,
(5) Addition products of from 15 to 60 mol of ethylene oxide to castor oil and/or hardened castor oil,
(6) Polyol and polyglycerol esters,
(7) Addition products of from 2 to 15 mol of ethylene oxide to castor oil and/or hardened castor oil,
(8) Partial esters based on unbranched, branched, unsaturated or saturated C_{6/22}-fatty acids, ricinoleic acid and also 12-hydroxystearic acid and glycerol, polyglycerol, pentaerythritol, dipentaerythritol, sugar alcohols, alkyl glucosides and also polyglucosides,
(9) Mono-, di- and trialkyl phosphates and also mono-, di- and/or tri-PEG-alkyl phosphates and salts thereof,
(10) Lanolin alcohols,
(11) Polysiloxane-polyalkyl-polyether copolymers and corresponding derivatives,
(12) Mixed esters of fatty acids containing from 6 to 22 carbons, methylglycose and polyols,
(13) polyalkylene glycols,
zwitterionic surfactants,
ampholytic surfactants,
quaternary emulsifiers,
glycerophospholipids and glyceroglycolipids, polysaccaarides (b-3) and/or
chelators (b-4), and
the carrier (C) being silica, and
the component (A) that is to be absorbed being mixed with a stabilizer (B) and subsequently applied to a carrier (C), wherein
the components (A) that are to be absorbed are conjugated octadecapolyenoic acids where less than 5% of the fatty acid content is 11,13-octadecadienoic acid isomers, 8,10-octadecadienoic acid isomers, cis,cis-octadecadienoic acid isomers or trans,trans-octadecadienoic acid isomers or mixtures of these isomers.

2. The process according to claim 1, wherein the mean particle size of the carrier (C) is at least 100 µm, preferably at least 200 µm.

3. The process according to any one of the preceding claims, wherein the amount of carrier (C) is greater than or equal to 10% by weight, based on the adsorbate.

4. The process according to any one of the preceding claims, wherein the ratio of stabilizer (B) to component (A) that is to be adsorbed is less than or equal to 10, in particular less than or equal to 5, in particular less than or equal to 1, preferably less than or equal to 0.8.

5. The process according to any one of the preceding claims, wherein the stabilizer (B) is at least one compound selected from the group consisting of the glycerides (b-1) and at least one compound selected from the group consisting of the emulsifiers (b-2).

6. The process according to any one of the preceding claims, wherein the adsorbate is coated.

7. The process according to claim 6, wherein the coating compound (D) is selected from the group consisting of
a) polyalkylene glycols, in particular polyethylene glycols, of a number-average molecular weight of from about 400 to 15 000,
b) polyalkylene oxide polymers or polyalkylene oxide copolymers of a number-average molecular weight of from about 4000 to 20 000, in particular block copolymers of polyoxyethylene and polyoxypropylene;
c) substituted polystyrenes, maleic acid derivatives and styrene-maleic acid copolymers;
d) polyvinylpyrrolidones of a number-average molecular weight of from about 7000 to 1 000 000;
e) vinylpyrrolidone/vinyl acetate copolymers of a number-average molecular weight of from about 30 000 to 100 000;
f) polyvinyl alcohol of a number-average molecular weight of from about 10 000 to 200 000, polyvinyl phthalate;
g) hydroxypropylmethyl cellulose of a number-average molecular weight of from about 6000 to 80 000;
h) alkyl(meth)acrylate polymers and copolymers of a number-average molecular weight of from about 100 000 to 1 000 000, in particular ethyl acrylate/methyl methacrylate copolymers and methacrylate/ethyl acrylate copolymers;
i) polyvinyl acetate of a number-average molecular weight of from about 250 000 to 700 000, optionally stabilized with polyvinyl pyrrolidone;
j) polyalkylenes, in particular polyethylenes;
k) phenoxyacetic acid-formaldehyde resin;
l) cellulose derivatives, such as ethyl cellulose, ethyl methyl cellulose, methyl cellulose, hydroxypropyl cellulose, hydroxypropyl methyl cellulose, carboxymethyl cellulose, cellulose acetate phthalate;
m) animal, vegetable or synthetic glycerides;
n) animal, vegetable or synthetic waxes or chemically modified animal, vegetable waxes, such as beeswax, candelilla wax, carnauba wax, montan ester wax and rice germ oil wax, spermaceti, lanolin, jojoba wax, Sasol wax, Japan wax or Japan wax substitute;
o) animal and vegetable proteins, for example gelatin, gelatin derivatives, gelatin substitutes, casein, whey, keratin, soybean protein; zein and wheat protein;
p) mono- and disaccharides, oligosaccharides, polysaccharides, for example starches, modified starches and also pectins, alginates, chitosan, carrageenans;
q) vegetable oils, for example sunflower, thistle, cottonseed, soybean, corn germ, olive, rape (seed), linseed, olive tree, coconut, palm kernel oil and palm oil;
r) synthetic or semisynthetic oils, for example medium-chain triglycerides or mineral oils;
s) animal oils, for example herring, sardine and whale oils;
t) hardened (hydrogenated or partially hydrogenated) oils/glycerides, for example of the abovementioned oils, in particular hydrogenated palm oil, hydrogenated cottonseed oil, hydrogenated soybean oil;
u) varnish coatings, for example terpenes, in particular shellac, Tolu balsam, Peru balsam, sandarac and silicone resins;
v) fatty acids, both saturated and monounsaturated and polyunsaturated C6 to C24 carboxylic acids;
w) silicas.

8. The process according to any one of the preceding claims, wherein the adsorbate comprises further substances (E) selected from the group consisting of antioxidants, preservatives, binders and/or flavorings.

9. The process according to claim 8, wherein the further substances (E) are added together with components (A).

10. The process according to claim 8, wherein the further substances (E) are mixed with the carrier.

11. An adsorbate obtainable by one of the abovementioned processes.

12. The use of the adsorbates according to claim 11 in foods, in cosmetics preparations, in preparations of animal nutrition, in particular in premixes.

## Revendications

1. Procédé pour la préparation d'adsorbats appropriés à l'alimentation humaine ou animale ainsi qu'à des applications cosmétiques, dans lequel on applique sur un support (C) un composant (A) à adsorber avec utilisation d'au moins un stabilisant (B), la taille moyenne de particule du support (C) étant d'au moins 80 µm,
le stabilisant (B) étant choisi dans le groupe constitué par
des glycérides (b-1),
des émulsifiants (b-2) choisis parmi
des tensioactifs non ioniques choisis dans au moins l'un des groupes suivants
(1) produits d'addition de 2 à 30 moles d'oxyde d'éthylène et/ou 0 à 5 moles d'oxyde de propylène sur des alcools gras linéaires ayant de 8 à 22 atomes de carbone, sur des acides gras ayant de 12 à 22 atomes de carbone et sur des alkylphénols ayant de 8 à 15 atomes de carbone dans le groupe alkyle,
(2) mono- et diesters d'acides gras en C₁₂-C₁₈ avec des produits d'addition de 1 à 30 moles d'oxyde d'éthylène sur le glycérol,
(3) mono- et diesters de glycérol et mono- et diesters de sorbitanne avec des acides gras saturés et des acides gras insaturés ayant de 6 à 22 atomes de carbone et leurs produits d'addition d'oxyde d'éthylène,
(4) alkyl-mono- et -oligoglycosides ayant de 8 à 22 atomes de carbone dans le radical alkyle et leurs analogues éthoxylés,
(5) produits d'addition de 15 à 60 moles d'oxyde d'éthylène sur l'huile de ricin et/ou l'huile de ricin hydrogénée,
(6) esters de polyols et polyglycérol,
(7) produits d'addition de 2 à 15 moles d'oxyde d'éthylène sur l'huile de ricin et/ou l'huile de ricin hydrogénée,
(8) esters partiels d'acides gras en C₆-C₂₂ linéaires, ramifiés, insaturés ou saturés, d'acide ricinoléique ainsi que d'acide 12-hydroxystéarique et de glycérol, polyglycérol, pentaérythritol, dipenta-érythritol, alcools glucidiques, alkylglucosides ainsi que polyglucosides,
(9) phosphates de mono-, di- et trialkyle ainsi que phosphates de mono-, di- et/ou tri-PEG-alkyle et leurs sels,
(10) alcolanums,
(11) copolymères polysiloxane-polyalkyl-polyéther ou dérivés correspondants,
(12) esters mixtes d'acides gras ayant de 6 à 22 atomes de carbone, méthylglucose et polyols,
(13) polyalkylèneglycols,
des tensioactifs zwitterioniques,
des tensioactifs ampholytes,
des émulsifiants quaternaires,
des glycérophospholipides et glycéroglycolipides, des polysaccharides (b-3) et/ou
des agents chélateurs (b-4), et
le support (C) étant la silice, et dans lequel on mélange le composant (A) à adsorber avec un stabilisant (B) et ensuite on applique le mélange sur un support (C), **caractérisé en ce que**
le composant (A) à adsorber consiste en des acides octadécapolyénoïques dans lesquels moins de 5 % de la fraction acide gras consistent en isomères d'acide 11,13-oCtadécadiénoïque, isomères d'acide 8,10-octadécadiénoïque, isomères d'acide cis-,cis-octadécadiénoïque ou isomères d'acide *trans-,trans-*octadécadiénoïque ou mélanges de ces isomères.

2. Procédé selon la revendication 1, **caractérisé en ce que** la taille moyenne de particule du support (C) est d'au moins 100 µm, de préférence d'au moins 200 µm.

3. Procédé selon l'une quelconque des revendications précédentes, **caractérisé en ce que** la quantité du support (C) est supérieure ou égale à 10 % en poids, par rapport à l'adsorbat.

4. Procédé selon l'une quelconque des revendications précédentes, **caractérisé en ce que** le rapport du stabilisant (B) aux composants (A) à adsorber est inférieur ou égal à 10, en particulier inférieur ou égal à 5, en particulier inférieur ou égal à 1, de préférence inférieur ou égal à 0,8.

5. Procédé selon l'une quelconque des revendications précédentes, **caractérisé en ce que** comme stabilisant (B) on choisit au moins un composé dans le groupe des glycérides (b-1) et au moins un composé dans le groupe des émulsifiants (b-2).

6. Procédé selon l'une quelconque des revendications précédentes, **caractérisé en ce qu'**on enrobe l'adsorbat.

7. Procédé selon la revendication 6, **caractérisé en ce que** l'agent d'enrobage (D) est choisi dans le groupe constitué par
a) des polyalkylèneglycols, en particulier des polyéthylèneglycols ayant une masse moléculaire moyenne en nombre d'environ 400 à 15 000,
b) des polymères polyoxyalkylène ou copolymères de polyoxyalkylène ayant une masse moléculaire moyenne en nombre d'environ 4 000 à 20 000, en particulier des copolymères séquencés de polyoxyéthylène et polyoxypropylène ;
c) des polystyrènes substitués, des dérivés d'acide maléique et des copolymères styrène/acide maléique ;
d) des polyvinylpyrrolidones ayant une masse moléculaire moyenne en nombre d'environ 7 000 à 1 000 000 ;
e) des copolymères vinylpyrrolidone/acétate de vinyle ayant une masse moléculaire moyenne en nombre d'environ 30 000 à 100 000 ;
f) un poly(alcool vinylique) ayant une masse moléculaire moyenne en nombre d'environ 10 000 à 200 000, le polyphtalate de vinyle ;
g) une hydroxypropylméthylcellulose ayant une masse moléculaire moyenne en nombre d'environ 6 000 à 80 000 ;
h) des polymères et copolymères de (méth)acrylate d'alkyle ayant une masse moléculaire moyenne en nombre d'environ 100 000 à 1 000 000, en particulier des copolymères acrylate d'éthyle/méthacrylate de méthyle et des copolymères méthacrylate/acrylate d'éthyle ;
i) un poly(acétate de vinyle) ayant une masse moléculaire moyenne en nombre d'environ 250 000 à 700 000, éventuellement stabilisé avec de la polyvinylpyrrolidone ;
j) des polyalkylènes, en particulier polyéthylènes ;
k) une résine acide phénoxyacétique-formaldéhyde ;
l) des dérivés de cellulose, tels que l'éthylcellulose, l'éthylméthylcellulose, la méthylcellulose, l'hydroxypropylcellulose, l'hydroxypropylméthyl-cellulose, la carboxyméthyl-cellulose, l'acétate-phtalate de cellulose ;
m) des glycérides animaux, végétaux ou synthétiques ;
n) des cires animales, végétales ou synthétiques ou des cires animales, végétales modifiées chimiquement, telles que la cire d'abeille, la cire de candelilla, la cire de carnauba, la cire de lignite et la cire d'huile de germes de riz, le blanc de baleine, la lanoline, la cire de jojoba, la cire Sasol, la cire du Japon ou un substitut de cire du Japon ;
o) des protéines animales et végétales, comme par exemple la gélatine, des dérivés de gélatine, des substituts de gélatine, la caséine, le petit-lait, la kératine, la protéine de soja ; la zéine et la protéine de blé ;
p) des mono- et disaccharides, oligosaccharides, polysaccharides, par exemple les amidons, les amidons modifiés ainsi que les pectines, les alginates, le chitosane, les carraghénanes ;
q) des huiles végétales, par exemple l'huile de tournesol, de carthame, de graines de coton, de soja, de germes de maïs, d'olive, de (graines de) colza, de lin, d'olivier, de coprah, de palmiste (à huile) et l'huile de palme ;
r) des huiles synthétiques ou semi-synthétiques, par exemple des triglycérides à longueur moyenne de chaîne ou des huiles minérales ;
s) des huiles animales, comme par exemple l'huile d'harengs, de sardines et de baleiné ;
t) des huiles/glycérides durcis (hydrogénés ou partiellement hydrogénés) comme par exemple des huiles indiquées ci-dessus, en particulier l'huile de palme hydrogénée, l'huiles de graines de coton hydrogénée, l'huile de soja hydrogénée ;
u) des revêtements de laque, comme par exemple des terpènes, en particulier la gomme-laque, le baume de Tolu, le baume du Pérou, la sandaraque, et les résines de silicone ;
v) des acides gras, des acides carboxyliques en C₆-C₂₄ aussi bien saturés que mono- et polyinsaturés ;
w) des silices.

8. Procédé selon l'une quelconque des revendications précédentes, **caractérisé en ce que** l'adsorbat contient d'autres substances (E), choisies dans le groupe constitué par des antioxydants, des conservateurs, des liants et/ou des agents de sapidité.

9. Procédé selon la revendication 8, **caractérisé en ce qu'**on ajoute les autres substances (E) conjointement avec les composants (A).

10. Procédé selon la revendication 8, **caractérisé en ce qu'**on mélange les autres substances (E) avec le support.

11. Adsorbats, pouvant être obtenus selon l'un des procédés précités.

12. Utilisation des adsorbats selon la revendication 11, dans des produits alimentaires, dans des préparations cosmétiques, dans des préparations de l'alimentation animale, en particulier dans des prémélanges.
